# EUROPEAN PATENT APPLICATION

(11) **EP 4 497 382 A1**
(43) Date of publication of application: **29.01.2025**
(21) Application number: 23218304.6
(22) Date of filing: 19.12.2023
(51) Int. Cl.: A61B 5/16, A61B 5/00

(54) **SYSTEM AND METHOD FOR ASSESSING ANHEDONIA**

(30) Priority: 27.07.2023 EP 23188236
(71) Applicant: Max-Planck-Gesellschaft zur Förderung der Wissenschaften e.V., 80539 München (DE)
(72) Inventor: Spoormaker, Victor, 80805 München (DE); Brendler, Andy, 80804 München (DE)
(74) Representative: LKGLOBAL Lorenz & Kopf Patentanwalt Attorney at Law PartG mbB

(57) **Abstract**

The invention relates to a system (100) for assessing anhedonia, comprising a task component (101) configured to provide a reward task to a user; a capture component (102) configured to capture biomarker data of a biomarker of the user in response to the reward task; and a processing component (103) configured to generate a biomarker response profile based on the biomarker data. The invention further relates to a method for assessing anhedonia, comprising the steps of providing a reward task, capturing biomarker data of a biomarker of the user in response to the reward task; and generating a biomarker response profile based on the biomarker data.

## Description

### Field

The invention relates to a system and method for assessing anhedonia, specifically capturing a biomarker of a user in response to a reward task.

Anhedonia is the reduced ability or inability to experience pleasure or positive emotions. Anhedonia can be assessed by various methods, such as self-report questionnaires, behavioral tasks, and physiological measures.

Anhedonia is a core symptom of several psychiatric disorders. It is a common symptom of depression and other mental disorders, and it can impair the quality of life and functioning of affected individuals. However, measuring anhedonia is not a straightforward task, as it involves multiple subcomponents of reward processing, such as motivation, hedonic impact, and learning. Different methods have been developed to assess these aspects of anhedonia, such as self-report questionnaires, behavioral tasks, and neuroimaging techniques. Some of the most widely used questionnaires are the Snaith-Hamilton Pleasure Scale (SHAPS), the Temporal Experience of Pleasure Scale (TEPS), and the Chapman Anhedonia Scales. These instruments aim to capture the subjective experience of pleasure in various domains of life, such as social, physical, and aesthetic. Behavioral tasks, on the other hand, measure the objective performance of individuals in situations that involve reward anticipation, consumption, or feedback. Examples of such tasks are the Effort-Expenditure for Rewards Task (EEfRT), the Monetary Incentive Delay Task (MIDT), and the Probabilistic Reward Task (PRT). These tasks can also be combined with neuroimaging techniques, such as functional magnetic resonance imaging (fMRI) or electroencephalography (EEG), to examine the neural correlates of reward processing in anhedonia. By using a multimodal approach, a more comprehensive understanding of the mechanisms underlying anhedonia and its impact on various psychiatric disorders can be gained.

A great number of cognitive and affective processes across species involve the locus coeruleus (LC) - the primary source of noradrenergic (NA) signaling in the brain. Pupillometry, an accessible surrogate marker of LC activity, is becoming increasingly attractive. The LC-NA system is directly affected by commonly prescribed antidepressants, which suggests the usefulness of pupillometry for treatment allocation and early response monitoring within subgroups of patients with major depressive disorder (MDD).

Depression can alter the pupillary light reflex, which is an index of pupil size in response to light, characterized by an immediate constriction followed by a dilation. Patients with a MDD diagnosis can have a slower constriction velocity and a smaller constriction change of the pupil in response to light stimuli compared to control participants. Smaller pupil dilation as a response to positively and negatively valenced face stimuli can predict depressive symptoms, e.g., following a natural disaster, and, compared to control participants, both larger or smaller pupil dilation to negatively valenced face stimuli can predict recurrence of depressive episodes. A larger pupil dilation response to sad faces can occur in children with a family history of depression. Furthermore, smaller pupil dilation in response to negative prediction errors in the feedback phase of a reward learning task can be associated with more depressive symptoms.

In addition, the prediction error-related pupil dilation can be inversely related to concentration levels of the neurotransmitter choline in the dorsal anterior cingulate, a core brain region of the salience network, which has been implicated in a range of mental disorders including depression. In this context, MDD patients can have smaller bilateral cerebral blood flow increases in anticipation of congruent and incongruent trials in a Stroop task and in anticipation of calculations in a mental arithmetic task, indicating differential anticipatory and preparatory processes when completing tasks.

A lack of arousal regulation can manifest itself in a reduction of the pupillary response. Pupil dilation during reward anticipation can be associated with activity in the dorsal anterior cingulate cortex and bilateral anterior insular cortex, that as an ensemble constitute the major hubs of the salience network. A negative correlation with depressive symptom load can be observed for regions in the salience network. This can be interpreted as a failure to maintain and upregulate arousal when preparing for the behavioral response to achieve reward in depression.

One of the physiological measures that has been shown to be sensitive to anhedonia is the pupil response. The pupil is a dynamic indicator of arousal, attention, and cognitive processes, and it can reflect changes in emotional states and reward processing. The pupil can dilate more in anticipation of larger rewards, and this dilation is correlated with the subjective value of the reward. Moreover, the pupil dilation during reward anticipation is reduced in patients with depression and other disorders characterized by anhedonia, and it is negatively associated with the severity of depressive symptoms and anhedonia. Therefore, the pupil response can be used as a reliable and objective marker of anhedonia and reward dysfunction.

### Summary

The present invention relates to a system for assessing anhedonia by capturing the pupil response when a reward task is provided. The system comprises a device for presenting visual stimuli to a subject, a device for measuring the pupil size of the subject, and a device for analyzing the pupil data. The reward anticipation task involves presenting the subject with cues that indicate the probability and magnitude of receiving a reward, preferably a monetary reward, or a neutral outcome, followed by the outcome feedback. The system captures the pupil response before, after and during the cue and the delay phases, and computes the pupil dilation as a function of the expected reward value. The system then compares the pupil dilation of the subject with a normative data set obtained from healthy controls, and calculates an anhedonia score based on the deviation from the norm. The anhedonia score can reflect the degree of impairment in reward anticipation and hedonic function of the subject, and can be used for diagnosis, monitoring, and treatment evaluation of anhedonia and related disorders. The system provides a novel, non-invasive, and cost-effective way of measuring anhedonia and reward processing in clinical and research settings.

According to a first aspect the invention relates to a system for assessing anhedonia, comprising a task component configured to provide a task, in particular a reward task to a user, a capture component configured to capture biomarker data of a biomarker of the user in response to the task, and a processing component configured to generate a biomarker response profile based on the biomarker data.

A biomarker response, e.g., pupil dilation, during reward anticipation can be associated with activity in the dorsal anterior cingulate cortex and bilateral anterior insular cortex, that as an ensemble constitute the major hubs of the salience network. The extent of pupil dilation during reward anticipation, e.g., a 6-second-long interval between the onset and offset of a reward-predicting cue, can be negatively correlated to depressive symptom load. A similar negative correlation with depressive symptom load can be observed for regions in the salience network. This can be interpreted as a failure to maintain and upregulate arousal when preparing for the behavioral response to achieve reward in depression. This process can be utilized to predict the subjective phenomenon of anhedonia.

The capture component may comprise an eye-tracking device which can be configured to measure the user's pupil dilation during or after the completion of the task. The processing component may be configured to receive data relating to the pupil dilation from the eye-tracking device and to analyze the data using one or more algorithms or models that are based on the relationship between pupil dilation and LC activity. The processing component may be configured to output feedback to the user or a clinician based on the anhedonia severity and/or the presence or absence of anhedonia. In one embodiment, the capture device, in particular comprising the eye-tracking device may be integrated in a smartphone or a virtual reality (VR) headset or augmented reality (AR) device. The user may download an application that provides an interface to the task component or the task component itself. The task component can provide various reward and control tasks. The user can perform the tasks on the smartphone screen, in particular while the smartphone is disposed at a fixed distance from the user's eyes. The smartphone can be arranged in a head gear mounted to the user's head. Alternatively, the smartphone can be set on a table. The task component can be configured to present a task, in particular present the task visually and/or auditorily to the user. The smartphone or VR headset (or AR device) may comprise the capture component in form of a camera which can be configured to capture images of the user's eyes and measure their pupil dilation using image processing techniques. The mobile device may comprise the processing component in form of a processor that can be configured to analyze the biomarker data, specifically pupil dilation data.

The task component can comprise a user interface, wherein the user interface is configured to capture a user input and provide the user input to the processing component. The user input can relate to a task response, i.e., correspond to a response requested by the task. The user interface can be configured to provide the task to the user in a perceivable manner, i.e., visually, auditorily and/or haptically. The user interface may be configured to provide feedback to the user based on a task response. Thereto, the user interface can be configured to acknowledge a user input by providing a confirmation signal, i.e., sound, visual indicator and/or haptic feedback signal (vibration). The user interface may further be configured to structure a task, respectively task conditions by providing a feedback signal. Thus, task conditions and/or tasks can be indicated to the user. The user interface can be configured visually, auditorily or haptically capture a user input. For example, the user can voice a feedback, look at a specific element, press a button and/or provide a touch response. The user interface can comprise a graphical interface component providing a platform interface to the user, wherein the platform interface can provide control functions to the user. Control functions can relate to at least one of the following functions: initiate a task, initiate various stimuli, initiate biomarker capture, initiate task performance analysis, initiate storage of task results and/or analysis results etc.

The processing component can be configured to utilize machine learning algorithms or neural networks that are trained on data from healthy and/or impaired individuals. The smartphone application can provide feedback to the user or a clinician based on the level of anhedonia, such as a score, a graph, a message, or an alert. The feedback can be generated remotely, i.e., by a server which may also comprise the processing component or perform a subset of functions associated with the processing component.

In another embodiment, the capture component, preferably comprising a display device and the eye-tracking device can be integrated in a VR headset or AR glasses. The user can wear the VR headset and experience various immersive environments that provide reward tasks, such as time critical response tasks. Additionally, all tasks providable via the task component, respectively a standard visual or sound output can be provided via the VR headset. The VR headset can measure the user's pupil dilation using specific capture components, preferably infrared sensors and/or cameras that can be embedded in the headset. The VR headset can communicate with the processing component which analyzes the pupil dilation data using statistical methods or artificial intelligence techniques that are based on the physiological principles of pupillometry. The VR headset can provide feedback to the user or a clinician based on the level of anhedonia, such as an audiovisual cue, a notification, or a report. The processing component can be provided by a cloud service.

The assessment of pupil responses to reward tasks can in particular identify a deviation from a baseline level of responses. The baseline level can be established by evaluating a plurality of non-pathological individuals, i.e., a suitable control group. An altered pupil response to reward may reflect anticipatory hypo-arousal, i.e., deviation from a predetermined norm can be associated with psychopathology across multiple disorders Furthermore, such baseline and reward-anticipatory changes in pupil responses can be predictive of a treatment response in pharmaco- and psychotherapy.

The biomarker, in particular the pupil size, dilation and change-points, can be captured task-evoked constituting a physiological readout for various forms of arousal, including reward-anticipatory arousal, which may represent the subjective symptom of anhedonia. The system according to the present invention can provide pupil response profiles during reward processing of a user. Herein, profiles of a heterogenous sample including healthy and unmedicated users can be used to establish a baseline for identifying pathological biomarker response profiles.

A relationship between a neurocognitive pathology, specifically anhedonia and pupil dilation can vary based on the severity. The system can be used to detect and/or diagnose a plurality of neuropsychiatric disorders and/or diseases. The system can be configured to determine anhedonia (i.e., currently the subjective symptom) and/or anticipatory hypo-arousal (the proposed underlying physiological process) of a user which can be used as an indicator across psychiatric diseases. Specifically, the system can be configured to determine a decline in hedonic functioning. Thereto specific pupillometric protocols can be used. The present system can provide a diagnosis based on the evaluation of the captured biomarker response profile, specifically the pupil size. The task component can be configured to periodically provide a task to the user and/or provide a reminder to the user to complete a task, respectively a task session, comprising a plurality of tasks. Herein, the system can be configured to monitor a progression of the anhedonic state of the user. This can achieve the advantage that impact of a medication can be determined, and/or that a threshold level to initiate medication can be monitored. This can achieve the advantage of increasing the effectiveness of a medication and thereby prognosis of the user as the medication may have an increased effectiveness in earlier stages of a psychiatric disease.

The modules can be integrated to form a single system component. Alternatively, the system can be modularized and/or decentralized. Specifically, the capture component can be in range to capture the biomarker from the user. The task component can be at a first remote location and can be configured to provide task data to a local interface configured to interact with the user to provide the task. Additionally and/or alternatively, the processing component can be at a second remote location. This can achieve the advantage that components relating to capturing the biomarker and presenting the affective task can be general purpose items, e.g., a smartphone, a computer or a VR-headset and the specialized task component and processing component can be located elsewhere.

The capture component and/or the processing component can form a high-resolution pupillometry system that can capture small changes in pupil diameter and account for factors such as eye movements, blinks, or head position. This achieves the advantage of capturing pupil response accurately and reliably. The capture component can comprise video-based eye trackers, infrared cameras, and/or wearable devices.

The processing component can be configured to determine a profile parameter based on the biomarker response profile and to assess anhedonia, preferably a severity of anhedonia, a presence or absence of anhedonia, basic arousal and/or anticipatory arousal, of the user based on the profile parameter.

This can achieve the advantage that the amount of data necessary for evaluation can be reduced. Specifically, the profile parameter can be a condensed representation of the biomarker response profile, i.e., specifying a key characteristic of the profile. The profile parameter can be assessed on a scale. The processing component can be configured to determine a z-score based on the biomarker data, respectively the biomarker response profile. Preferably, the pupillometry data can be z-scored, comprising standardizing with a mean value or the standard deviation. Thereby, observations from different distributions that may have different units or scales can be compared. The processing component can be configured to identify outlier values that are unusually high or low compared to the rest of the data. Herein, the z-score may be used to errors, and/or anomalies in the biomarker data, respectively biomarker response profile. The processing component can be configured to determine probabilities and percentiles using the standard normal distribution. For example, the processing component can be configured to determine a probability of a user having pathologic impairment, respectively psychopathological or neurological disease. A z-score can represent an efficient measure to analyze the biomarker response profile, since only the mean and/or standard deviation of the biomarker response profile may need to be calculated. Z-scores can be a measure that enables efficient comparison between different datasets as they have a mean of zero and a standard deviation of one. Also, z-scores can be compared within a single biomarker response profile by looking at their relative positions and/or values corresponding to the various stimuli (differential responses).

Preferably the scale can comprise threshold values relating to the potential of the profile parameter indicating a pathology. The profile parameter can be provided separate from the reward task and/or separate from the stimulus, in particular to a review component configured to provide diagnostic data based on the biomarker data, respectively biomarker response profile, preferably in relation to the stimulus or a plurality of stimulus responses performed by the user. Herein, the plurality of stimuli can be immediately consecutive, i.e., without a significant time delay. For example, the tests can be continuous, in particular such that the type of tests remains the same but various control stimuli can be added. One stimulus can e.g. predict an upcoming possibility to receive a monetary reward, another to receive positive verbal feedback, and yet another no outcome. Additionally or alternatively, the stimulus types can be provided at different times, e.g., with time intervals of minutes, hours or days.

The profile parameter may correspond to a statistical measure and/or a parameter of a mathematical function, in particular a function fitted to the biomarker response profile. The processing component can be configured to automatically determine the statistical measure by processing the biomarker response profile. The statistical measure can be based on a mathematical model, wherein parameters of the mathematical model can correlate to specific properties of the biomarker, biomarker data, respectively biomarker response profile. For example, fitted parameters according to the statistical measure can correlate to a pupil parameter: pupil size, pupil size variance, latency of pupillary constriction to light, constriction amplitude (decrease or increase), redilation after light offset, maximum velocity of constriction (MCV) and/or maximum constriction acceleration (MCA).

Furthermore, the processing component can be configured to determine a mean size, a mean dilation, i.e., a mean increase rate of the pupil, an acceleration of the dilation, a median dilation, a mean acceleration of the dilation, and/or a median acceleration of the dilation. Additionally or alternatively, the mean, median and/or variance of pupil size across a time interval, preferably a preferably a time interval of 1 to 10 seconds, more preferably of 4 to 8 seconds, even more preferably a time interval of 6 seconds, can be determined. The processing component can be configured to associate a biomarker response profile as a response to target stimuli, including the temporal windows before and after the relevant stimuli. A biomarker threshold model can be used to assess the effects of the captured biomarker, in particular on determining an arousal, hypo-arousal, respectively anhedonia. A biomarker response profile can be a time-vector of biomarker values that reflects the response of the user to a stimulus. Fitting the biomarker response profile can help to identify threshold values and/or time intervals of significance.

Furthermore, the processing component can be configured to fit the biomarker response profile linearly or non-linearly. The fitting process can estimate the parameters of the chosen fitting model, e.g., baseline hazard function, regression coefficients, and/or threshold parameter. The threshold parameter can represent optimal interval separations for fitting and increase fitting precision. The fitting process can also provide confidence intervals and hypothesis tests for the model parameters, as well as measures of model fit and goodness-of-fit.

The task component can comprise a presentation module configured to provide a stimulus perceivable by the user. The presentation module can provide the task visibly and/or auditorily. The presentation module can be provided to the user to enhance task focus. The task module can also provide guidelines on how to perform the task through the presentation component.

The stimulus can comprise at least one of a geometric pattern, an image, preferably an image of an object, a symbol, and/or an auditory stimulus. The stimulus can function as a signal to the user which consequence to expect when the task is completed successfully. Preferably, the consequence is a reward or the lack of a reward. The image can comprise a geometric pattern and/or shape. In particular, when providing a plurality of stimuli, each stimulus is substantially different from the other stimuli to render each stimulus easily differentiable and/or recognizable by the user. For example, different types a similar pattern, i.e., black and white stripes, can be used. Each pattern can be associated with an expected consequence. The stimuli can be differentiated by image complexity, e.g., the stimuli can differ in image complexity.

The task component can be configured to provide the stimulus for a predetermined time interval, in particular wherein the stimulus has a duration of at least 1 second, preferably a duration of 4 to 8 seconds, more preferably a duration of 6 seconds. The stimulus can be presented continuously, in particular until a stimulus response by the user is required to complete the task. The time interval for the stimulus response to occur can be initiated by a trigger signal. The time interval for which the stimulus is present can be an anticipation interval during which the user is primed to respond to the trigger signal. In particular, the user can be prepared to respond to the trigger signal as quickly as possible. The anticipation of the trigger signal can have a significant impact on the biomarker. The time interval of the stimulus can be adjusted to maximize a biomarker response difference of users having varying levels of anhedonia severity.

The task component can be configured to provide a plurality of stimuli, preferably to provide a set of stimuli comprising up to 15 stimuli. Within the set of stimuli a specific stimulus can be repeated to increase the signal-to-noise ratio. Repetition of a stimuli may not count towards the number of stimuli within a set. Each stimulus may be connected to a different reward, i.e., inducing different levels of reward anticipation. Preferably, each stimulus can be repeated preferably 5 to 10 times. Each repetition can be linked to a predetermined stimuli response. A correlation of hypo-arousal and the biomarker can be determined with a single stimulus, in particular a reward stimulus.

Each stimulus of the plurality of stimuli, and/or the differential responses among stimuli can provide predictive information about anhedonia (by means of basic and stimulus-induced arousal).

The presence or absence, and/or the severity of anhedonia can be dependent to various of the above-mentioned characteristics of the pupil response to the plurality of stimuli, presumably by capturing the anticipatory hypo-arousal response of the user. Anticipatory hypo-arousal can be reflecting a reduction in arousal, respectively basic versus induced excitement by the stimuli of the user.

The task component can be configured to provide a trigger signal. The trigger signal can differ substantially in perception by the user with respect to the stimulus. The stimulus is configured to prime the user for the stimulus response to be performed. Typically, the stimulus is provided over an extended time interval during which the biomarker response is captured. In contrast, the trigger signal is provided for a substantially shorter duration, i.e. only as a pulse signal with brief duration. This trigger signal and subsequent response time can, besides signaling the response requirement, provide an additional biomarker window-of-interest in combination with the above-mentioned pupil response characteristics. The stimulus can be presented in such a manner that its perception has no or minimal effects on the biomarker (no change in light intensity), the trigger signal can be presented as a clearly visible and different stimulus, such as a light flash, a loud noise or contain another salient sensory modality.

The task component can be configured to provide the trigger signal after the stimulus, preferably directly after the stimulus or after a brief pause, wherein the stimulus is not provided during the pause. The brief pause can have a time duration perceivable by the user.

The trigger signal or the end of the trigger signal can define the start of a response interval in which a stimulus response by the user is captured. This can be used to assess the success of the user completing the task, e.g., providing the stimulus response within a predetermined time interval or at a specific time after the trigger signal. For example, the task could be completed successfully and thereby result in a reward, when the stimulus response is provided according to the predetermined task condition, e.g., providing the response as fast as possible. The task component can also be configured to provide a set of stimuli and/or a set of trigger signals as a single task. For example, the stimuli can be followed by a set of trigger signals. The trigger signals can be separated by a predetermined time interval. Each trigger signal may require a stimulus response. Task conditions can be set accordingly. For example, from the set of trigger signals a predetermined number of stimulus responses should be performed within a given or performance-adjustable time interval, or alternatively all stimulus responses should be performed within the given time interval. Adjusting according to performance can relate to a reaction time of the user, e.g., when a response to the trigger signal is performed within a first time interval, the success condition can be altered such that the response to a subsequent trigger signal must occur within a second time interval which is shorter than the first time interval. For example, the first time interval can be 350 ms, and the second time interval can be 300 ms. Additionally and/or alternatively, the time interval can be increased should the response to the trigger signal not occur within the first time interval.

The capture component can be configured to capture a stimulus response. The stimulus response can be initiated by the trigger signal being provided. The stimulus can be defined as an action or generally any discernible willing action by the user to be captured by the capture component. The capture component can be configured to continuously capture an input signal associated with the stimulus response during the task. Thus, any capturable action by the user can be recorded. For example, a stimulus response prior to the trigger signal and/or during the subsequent stimulus can be captured. A trigger signal being captured within a predetermined time interval can correspond to a successful completion of a task condition. This can result in a reward being provided to the user and/or providing information of the successful completion to the user.

The trigger signal can comprise a visual and/or auditory signal, in particular a light flash. Preferably, the same device as is used for providing the stimulus is also used for providing the trigger signal. The trigger signal can be provided while the stimulus is still provided or preferably subsequent to the stimulus being provided. The trigger signal can be a combination of a visual, auditory, haptic and/or sensory signal. Haptic signals can be a vibration, pressure, movement or rotation exerted onto at least part of the user and/or a respective trigger device being in contact with the user. The trigger signal may also comprise a neuro-active component, i.e., providing a temperature signal and/or neurologically perceivable signal to the user. The neurologically perceivable signal can be an electrical signal perceivable by nerves and/or the central nervous system of the user. The trigger response signal can be chosen to minimize a response delay by the user. Additionally and/or alternatively, the trigger signal can be chosen to achieve a predetermined response delay by the user.

The stimulus response can comprise at least one of
a motor response;
an auditory, respectively verbal response;
an expression response; and/or
a neurological response.

A motor response can be performed by a muscle activation by the user. The motor response may be captured directly or indirectly by the capture component, i.e., a button can be pressed or a movement of the user can be captured by a camera module. The stimulus response can be predefined and known to the user. The required stimulus response can be linked to the type of stimulus being provided, i.e., a specific geometric pattern requires a specific response, i.e., a button press, or a choice between one of two button presses.

The capture component can be configured to capture a stimulus response for each provided stimulus. For each stimulus an expected stimulus response by the user can be defined. Providing the stimulus and capturing the stimulus can be subsequent steps. Stimuli can also explicitly not require a response.

The processing component can be configured to assess the stimulus response based on the provided stimulus according to a set of task rules. The task rules can determine the type of stimulus response required and/or the timing conditions on providing the stimulus response. Assessing the stimulus response can comprise determining the time delay between the trigger signal and the stimulus response being provided, determining whether an acceptable stimulus response was performed. A specific stimulus response or a plurality of stimulus responses can be defined as acceptable according to the task rules.

Assessing the stimulus response can comprise matching the stimulus response to the stimulus. Matching can comprise associating a captured stimulus response to a specific stimulus, i.e., the captured stimulus response is marked as an intended response to a specific stimulus. This can achieve the advantage that the processing component can determine which stimulus response is linked to which stimulus. In particular stimulus responses falling outside of predetermined response windows can be associated with a specific stimulus.

The processing module can be configured to determine a response time, preferably a time interval between the stimulus and the stimulus response by the user. Alternatively, the response window can be defined as a time interval following the trigger signal. In particular a time interval starting with the end or start of the trigger signal and the end or start of the stimulus response. The response time can be compared to a task rule, wherein the outcome of the comparison to the task rule can determine the provision of a reward. The result of the task rule comparison can be provided to the user, in particular provided immediately. A threshold for a maximum response time can be set to determine an acceptably fast stimulus response which in turn qualifies for a reward. The maximum response time can be adjustable, in particular adjustable to a responsiveness, respectively response capability of the user. Herein, a response capability can be a fastest response time the specific user is capable of. The threshold can be in the range of 20 ms to 1000 ms, preferably 300 ms or 400 ms. Should the response time be exceeded by the user, the reward can be denied.

The processing component can be configured to match the stimulus response to the stimulus based on the time interval. This achieves the advantage that a stimulus response captured within the predetermined response window can be tracked as the response to the respective stimulus.

The system can comprise a reward module configured to provide a reward to the user based on a reward condition being met. The reward module can record rewards achieved by the user. The reward module can be configured to provide the reward directly and/or to provide cumulative rewards after the response is completed.

The reward module can be configured to provide a reward associated with a primary reinforcer. A primary reinforcer can be a stimulus that satisfies a biological need, such as food, water, and/or warmth. By providing a reward associated with a primary reinforcer, the motivation and/or well-being of the recipient can be increased. This can be useful when the user shows low interest in the provided task, as a primary reinforcer can be more effective than a secondary reinforcer, which is a stimulus that has acquired value through learning, such as money, praise, or grades. Additionally, a primary reinforcer can be used to establish a positive association with the respective stimulus or task in general.

The reward module can be configured to provide a reward associated with a secondary reinforcer. A secondary reinforcer can be a stimulus that has acquired value through learning and association with a primary reinforcer. For example, money is a secondary reinforcer that can be used to buy primary reinforcers such as food, clothing, and/or shelter. A secondary reinforcer can increase the biomarker response in comparison to using a primary reinforcer as it is not tied to biological needs. Should the user not experience a desire associated with a primary reinforcer, providing that primary reinforcer may not be an effective reward. Using a secondary reinforcer can be more versatile and flexible than primary reinforcers because it can be exchanged for different types of rewards. For example, money can be used to buy goods and services, depending on the preferences and needs of the user. Similarly, tokens can be used to access different activities or privileges in a token economy system. Using a secondary reinforcer can be more convenient and/or efficient than primary reinforcers because it can be delivered quickly and easily.

The reward condition can comprise at least one of the following: the time interval not exceeding a maximum threshold value; the stimulus response corresponding to an expected stimulus response type; a stimulus response pattern corresponding to an expected stimulus pattern (e.g., a similarity to norm response); and/or the stimulus response adhering to the task rules. The task rules can be provided to the user prior to being exposed to the stimuli, respectively prior to the tasks starting. Stimulus response requirements and/or rewards can thus be known prior to performing the task, respectively prior to responding to the stimuli. The task rules can link a specific stimulus to a specific reward condition (reward, no reward, no stimulus response required etc.). The task rules can further specify conditions pertaining to the stimulus response, i.e., which action, i.e., user response, the stimulus response should comprise, by which means the action can be performed and/or under which time constraints the action shall be performed.

The reward module can be configured to provide at least one of the following reinforcers:
- a primary reinforcer;
- a secondary reinforcers; such as a symbol or image; a monetary reward, preferably an auditory and/or visual confirmation of the acquisition of the monetary reward by the user; and/or a predetermined number of points.

The task component can be configured to provide the reward condition as part of the task to the user, preferably prior to the task being provided. The reward condition can define a success state and/or a failure state. The success state can be coupled to the reward being provided. The failure state can be coupled to a reward being denied. The reward condition can establish a limit to a stimuli response qualifying for a success state. The success state and/or failure state can be indicated to the user.

The task can comprise a reward sequence for capturing biomarker data in relation to an anticipated reward and/or a control sequence for capturing biomarker data in relation to anticipating the lack of a reward and/or a passive sequence for capturing biomarker data in relation to the lack of a trigger. A reward sequence can comprise at least one stimulus associated with the potential for receiving a reward. The reward sequence can comprise at least one trigger signal prompting the user to provide a stimulus response. The control sequence can comprise at least one stimulus associated with no potential for receiving a reward. The control sequence can comprise at least one trigger signal prompting the user to provide a stimulus response. For the control sequence the user is prompted for a stimulus response and an indication of whether the stimulus was provided according to the task rules can be given but no reward will be issued. The user may be aware of the control type and that no reward can be gained.

The reward sequence can comprise a reward stimulus, wherein the reward stimulus indicates the potential for receiving a reward to the user.

The reward sequence can comprise a reward trigger at a predetermined time interval after the reward stimulus.

The task component can be configured to provide the reward trigger within a predetermined time interval after the reward stimulus.

The processing component can be configured to apply a predetermined control reward condition when processing the captured stimulus response, preferably wherein the predetermined control reward condition comprises a response time captured below a maximum response time threshold value. Meeting the control reward condition can mark the stimulus response as successful. When the reward sequence is captured a successful stimulus response can trigger providing a reward and a success indicator. When the control sequence is captured a successful stimulus response can trigger providing a success indicator only.

The control sequence can comprise a control stimulus. The control stimulus can indicate that there is no potential for the user to receive a reward, or to receive a reward of reduced value, e.g. receiving positive verbal feedback in comparison to a monetary reward.

The control sequence can comprise a control trigger signal at a predetermined time after the control stimulus. The control trigger signal can prompt the user to provide a stimulus response. The control trigger signal can be the same signal as the trigger signal.

The task component can be configured to provide the control trigger within a predetermined time interval after the control stimulus. For providing the control trigger the same conditions as applying the trigger signal can apply.

The passive sequence can comprise a passive stimulus, wherein the passive stimulus indicates the absence of a trigger signal and that there is no potential for the user to receive a reward.

The passive sequence can comprise a trigger signal. The trigger signal can be provided after the passive stimulus. The passive stimulus can indicate to the user that the trigger signal will follow. Herein, the passive stimulus may also indicate that there is no potential for the user to receive a reward. For example, up to four types of stimuli can be used, a reward stimulus, a control stimulus, a passive stimulus with subsequent trigger signal and/or a passive stimulus without a subsequent trigger signal.

The task component can be configured to provide an identical signal as the response trigger and the control trigger.

The task component can be configured to provide a first stimulus as the reward stimulus and a further stimulus as the control stimulus. The first stimulus and the further stimulus can be distinguishable with respect to one another by the user. In particular the perceivable presentation of the stimuli can be readily distinguishable. This achieves the advantage that the user is aware of the type of stimulus and the associated reward conditions within a negligible amount of time after being provided with the stimulus.

The reward stimulus, the control stimulus and/or the passive stimulus can be impact neutral with respect to the biomarker data, biomarker response profile and/or profile parameter when being perceived by the user without reward context. Being impact neutral can be defined as having no measurable effect on the captured biomarker. In particular when no further meaning is associated with the respective stimulus. For example, when capturing the pupil diameter as the biomarker and providing the stimuli visually, the pattern, brightness, contrast and/or shape of the stimuli can be chosen such that changing the stimuli, going from providing the stimuli, to not providing the stimuli or vice versa does not measurably change the pupil diameter. This can increase the effect of the assigned reward condition of the stimuli on the biomarker and thus increase the quality of the captured signal. In particular, effects on the biomarker not related to the anticipated reward possibilities can be minimized.

The reward stimulus, the control stimulus and/or the passive stimulus can be geometric transformations with respect to one another, in particular when being perceived by the user without reward context. The stimuli can be based on the same geometric shape or pattern. For example, the pattern can be rotated, flipped, stretched or warped. Thus the different stimuli may be similar in their overall appearance but have a substantial visual difference to be directly discernible from one another. The time interval for the stimuli being presented to the user can be fixed. This time interval can represent a reward anticipation phase. Herein a direct distinction between the stimuli provides a consistent time interval length in which the stimuli are being perceived. In other words, the time interval of the stimuli being perceived is not significantly reduced by the user identifying the stimuli. In other words, the stimulus and its meaning are easily perceivable and/or recognizable by the user. This can achieve the advantage that there is no significant time delay in the anticipation window during which the user tries to decipher the stimulus. When measuring the response interval, a delay due to perception can be considered.

The reward stimulus, the control stimulus and/or the passive stimulus are defined within a predetermined biomarker response value interval.

The capture component can be configured to apply a filter, in particular a spatial filter, to the reward stimulus, the control stimulus and/or the passive stimulus. The filter can be configured to modify the reward stimulus, in particular modify the user perceivable signal representation of the stimulus. For example, a binary geometric color representation can be filtered to mitigate contrasts that could have a significant effect on the biomarker. The filter can also be a frequency filter, i.e., acting as a blurring or band filter. Additionally, the filter can comprise a low pass or high pass. The filter can alter the perception of the stimulus by the user.

The capture component can be configured to determine and/or to remove effects of the reward stimulus, control stimulus and/or the passive stimulus from the biomarker data, preferably to remove signal cross-contamination from the stimulus into the biomarker data. Perceiving the stimulus itself can impact the biomarker in addition to the anticipated arousal impact. This impact can be determined by providing stimuli having a comparable impact when perceived by the user but not being associated with a reward anticipatory impact.

The task component can be configured to provide a segmented task comprising at least one of: an anticipation phase, which preferably comprises the reward stimulus or the control stimulus; a response trigger phase; a response capture phase; a feedback phase; a reward consumption phase; and/or a fixation phase.

Preferably, a task can comprise a sequence of an anticipation phase during which a stimulus is perceived. The anticipation phase can be followed by a response trigger phase during which a trigger signal is provided. The response trigger phase can be long enough to render the comprised trigger signal perceivable by the user and/or short enough to not provide a significant time window between the anticipation phase and the response capture phase, although an alternative stimulus presentation procedure with a pause in between the stimulus and the trigger is optional. The response capture phase can thus almost immediately follow the anticipation phase. The response capture phase can be a time interval during which a stimulus response is expected, in particular during which the stimulus response can be captured by the capture component. The feedback phase can follow the response capture phase. The task component can be configured to provide a feedback signal pertaining to the success or failure status of the captured stimulus response in relation to the provided trigger signal and/or stimulus. The feedback signal can be provided during the feedback phase. The task component can be configured to provide the reward and/or to provide an information relating to obtaining the reward in the reward consumption phase.

The capture component can be configured to capture biomarker data during at least one phase of the segmented task, preferably during each phase of the segmented task.

The capture component can be configured to synchronize capturing the biomarker data with the reward stimulus, preferably such that a time interval before, during and/or after the reward stimulus is captured. This can achieve the advantage that the biomarker can be captured for the complete duration of the stimulus. The captured biomarker data can be sequenced according to the stimulus, trigger signal, reward information being displayed and/or the reward being consumed, as well as in the following fixation phase before the subsequent stimulus. Processing of the biomarker data can differ based on the segment of the task. Biomarker data captured prior to providing the stimulus can be used for baseline correction. Biomarker data captured after providing the stimulus can be used to determine reward effects. Biomarker data captured during the inter-stimulus-interval can be used to determine recovery/baseline effects.

The capture component can be configured to synchronize capturing the biomarker data with the control stimulus, preferably such that a time interval during the secondary control stimulus can be captured. The processing component can be configured to compare biomarker data captured during a control stimulus with further biomarker data captured during a reward stimulus to determine anhedonia presence and/or severity of the user, by means of comparing various pupil response characteristics within and among stimuli.

The biomarker data can comprise a biomarker vector, preferably a pupil size vector. The biomarker vector can be a time series of captured biomarker values. The biomarker data can be binned and averaged. The biomarker data can be multi-dimensional, i.e., comprising a plurality of captured bio signals.

The processing component can be configured to determine a derivative of the biomarker vector, preferably a first derivative and/or a second derivative.

The processing component can be configured to apply a fit to at least part of the biomarker vector, in particular a linear or non-linear fit.

The processing component can be configured to segment the biomarker vector and to apply a fit to at least one segment of the biomarker vector. The processing component can be configured to apply differing fits to different segments of the biomarker vector. This can achieve the advantage that the type of processing can be adapted to the type of segment. The time interval during which the stimulus is perceived can be segmented according to an associated function of each segment, i.e., providing the stimulus, providing the trigger signal, stimulus response window. During each segment the biomarker can correlate to different functions, i.e. have different scaling behavior. Each functional interval can be binned further. For example, the stimulus perception can be segmented into a plurality of intervals, wherein each interval is fitted separately. The anticipation of the trigger signal at the end of or after the time interval during which the stimulus is presented, can affect the biomarker signal, i.e., the biomarker response can be increased.

The processing component can be configured to determine a profile parameter based on the fit, preferably to determine a fit characteristic of the biomarker vector. The fit characteristic can be an intercept value and/or an acceleration value. Depending on the fitted function, the fit characteristic can be any corresponding fit parameter.

The processing component can be configured to apply the fit time-bin-wise to the biomarker vector, preferably wherein each bin represents a predetermined time interval, in particular a time-interval between 0,5-2,0 s. The processing component can be configured to perform the binning uniformly or non-uniformly. A non-uniform binning can adhere to a specific predetermined expectation of quantitatively different biomarker responses.

The segment can correspond to at least one of the following:
- a first time interval starting with the reward stimulus or the control stimulus, preferably wherein the first time interval is binned, in particular binned in 1 or 2 s intervals;
- an onset time interval starting at a predetermined time, preferably between 100 ms and 2000 ms, more preferably 500 ms or 1000 ms before the reward trigger, respectively the control trigger;
- a finalizing time interval ending with the subsequent stimulus, preferably starting 500 ms and 5000 ms, more preferably 2000 ms before the subsequent stimulus;
- a predetermined segment between the stimulus and the subsequent stimulus;
- a full stimulus duration.

Providing the reward stimulus can define a reward anticipation window, i.e., time interval. This interval can have a predetermined, in particular fixed, duration. The user can preferably be aware of this duration. This time interval can be segmented, e.g., 1 second bins can be applied during a 6 second interval, or the full interval can be utilized.

The task component can be configured to repeat a task for a plurality of instances. This can allow for statistical processing of the repetitions to increase the signal quality, e.g., increase the signal-to-noise ratio or achieve a smoother signal.

The processing component can be configured to compare stimulus responses based on the plurality of captured biomarker responses corresponding to the respective task instances. Thereby, the processing component can achieve the advantage that differences between the stimuli responses captured by the biomarker data can be determined. The processing component can be configured to determine a level of anhedonia based on differences in stimuli correlated to different reinforcers and corresponding pupil responses to the respective predictive stimulus types.

The processing component can be configured to determine the profile parameter for each task instance and to compare the profile parameter across the task instances. The profile parameter can be an indicator for the overall response of the user to the task captured in the biomarker data. The comparison of profile parameters can increase processing efficiency or result processing, respectively result assessment by the user and/or a third party, e.g., a physician.

The processing component can be configured to determine a level of reward anticipatory hypo-arousal representing anhedonia, in particular by matching the profile parameter to a hypo-arousal scale, in particular a quantitative or qualitative scale.

The task component can be configured to repeat a task or a task component for a predetermined number of times. The processing component can be configured to statistically process the repeated task instances, respectively to corresponding biomarker profiles. Repeating a task can comprise providing the same task consecutively or providing a repetition sequence of a reward task, control task and/or passive task. A reward task corresponds to a task comprising a reward stimulus, a control task corresponds to a task comprising a control stimulus and a passive task corresponds to a task comprising a passive stimulus, i.e., a stimulus that is not followed by a trigger signal and no user response is required.

The task component can be configured to provide the repeated tasks or task components in a predetermined sequence, in particular in a predetermined sequence of stimuli, preferably repeating stimuli.

The system can comprise a machine learning component configured to train a machine learning model based on the biomarker training data. The machine learning model can be adapted to identify signal features that characterize anhedonia presence or absence and/or anhedonia severity in the user, presumably associated with anticipatory arousal. The machine learning model can be adapted to identify signal features characterizing anticipatory hypo arousal and/or reduced attention. These features, preferably the scaling of these features, can be mapped to the presence or absence of anhedonia and/or the severity of anhedonia. For the example of the biomarker being the pupil size, a reduced pupil response can be linked to reward anticipatory hypo-arousal and/or reduced attention. The system can be trained based on the biomarker data, which can be collected from a large and diverse sample of users. Thereby, the machine learning component can generalize to new and unseen biomarker data. This can improve the accuracy and reliability of the system in identifying anticipatory hypo-arousal, respectively anhedonia, in different users and contexts.

The processing component can be configured to apply the machine learning model to biomarker data and to output a level of anhedonia of the user based on the signal features. The machine learning module can adapt the machine learning model to specific signal features that are relevant for anticipatory hypo-arousal and anhedonia, which can vary depending on the type, source, and quality of the biomarker data. This can enhance the robustness and flexibility of the system in handling different types of biomarker data and noise.

The machine learning model can be a neural network that comprises one or more hidden layers and an output layer. The output layer can provide a probability distribution over different levels of anhedonia. Thereby a neural network can be used to learn complex and nonlinear relationships between the biomarker data, in particular correlations of pathologies with specific biomarker data features, biomarker profiles and/or biomarker profile parameters. This can increase the performance and scalability of the system in identifying anticipatory hypo-arousal and anhedonia from high-dimensional and heterogeneous biomarker data. The neural network can comprise a support vector machine.

Based on the application of the machine learning model, the processing component can provide a probability distribution over different levels of anhedonia, which can reflect the uncertainty and variability of the prediction. This can improve the interpretability and usability of the system in providing meaningful and actionable feedback to the user and/or a clinician.

The machine learning component can be configured to perform a cross-validation on the trained machine learning model using a validation set of biomarker data, wherein the cross-validation comprises evaluating the performance of the machine learning model on the validation set and adjusting one or more parameters of the machine learning model based on the evaluation. Thereby, a cross-validation, i.e., a method to assess and improve the generalization ability of a machine learning model can be performed. This can reduce the risk of overfitting and/or underfitting, increasing the validity and applicability of the system.

The processing component can be adjustable to one or more parameters of the machine learning model based on the evaluation, which can optimize the performance and efficiency of the system in identifying anticipatory hypo-arousal and anhedonia. This can involve tuning hyperparameters of the neural network, such as the number of hidden layers, the number of neurons, the activation function, the learning rate and/or the regularization, etc.

The processing component can be configured to perform a feature extraction on the biomarker data. The feature extraction comprises applying one or more filters and/or transformations to the biomarker data to obtain a reduced set of features that are relevant for the anticipatory hypo-arousal identification. Thereby, a feature extraction can be performed, comprising transforming raw biomarker data into a more compact and informative representation that can capture the essential characteristics of the data. This can involve applying one or more filters and/or transformations, such as low-pass filtering, high-pass filtering, band-pass filtering, Fourier transform, wavelet transform, principal component analysis, etc.

The processing component can be configured to apply the machine learning model to the preprocessed biomarker data and to output a level of anhedonia in the subject based on the signal features. By applying the machine learning model to the pre-processed biomarker data, the dimensionality and complexity of the data can be reduced, noise and outliers can be removed, the signal-to-noise ratio can be enhanced, the computational speed and efficiency can be increased, and/or the accuracy and stability of the system can be improved.

The profile parameter can correspond to a statistical measure and/or a parameter of a mathematical function, in particular a function fitted to the biomarker response profile.

The processing component may be configured to determine a difference measure and to assess anhedonia presence and/or severity, and/or anticipatory arousal of the user based on the difference measure. The difference measure can be a Euclidean distance or a set of Euclidean distance of the biomarker response profile to a reference profile. The reference profile can be a statistical average of a plurality of biomarker response profiles. The reference profile can be associated with a healthy control group. Alternatively, the reference profile can be associated with a control group having a specific pathology. Furthermore, the difference measure can comprise a plurality of difference values, wherein each difference value is associated with a specific reference profile. Accordingly, not only the distance to a healthy control profile, but also the distance to a pathologic control profile, preferably specific pathologies, can be determined. A similarity, i.e., reduced distance to one of the control profiles, i.e., a similarity of the profiles, can indicate am an affiliation of the captured biomarker response profile to that specific reference profile. The processing component can be configured to assign the captured biomarker response profile to a specific reference profile, and thereby to a specific medical condition based on the difference measure.

The difference measure can correspond to at least one of the following:
difference of the profile parameter to a predetermined baseline value;
difference of the profile parameter;
differences among stimuli compared to the predetermined baseline differences;
difference of a fitted curve parameter to a predetermined fitted curve parameter;
similarity of the biomarker response profile to a reference profile;
scaling of the biomarker response profile in comparison to a reference profile;
scaling of a parameter fitted to the biomarker response profile in comparison to a reference parameter.

The processing component can be configured to determine an anhedonia measure based on the biomarker response profile and/or the difference measure. The anhedonia measure can be correlated with the anticipatory arousal response of the user. This can be a generalized measure based on biomarker response profile and/or the performance in the task, i.e., time to completion, errors, speed per individual component of the task, corrections etc. The processing component can be configured to provide the anhedonia measure to the user and/or to a medical data interface. This enables remote diagnosis and/or remote monitoring of the user's arousal function, in particular as a marker for neuropsychiatric and neurological pathologies. The processing component can be configured to assess the anhedonia measure in relation to previously determined anhedonia measures to determine an onset, a progression and/or improvement of a psychopathology.

The processing component can be configured to compare the difference measure and/or the biomarker response profile against a set of predetermined classification criteria (values). The processing component may further be configured to associate the biomarker response profile with an anhedonia class, which in particular is presumed to correspond to a pathological anticipatory arousal impairment of the user. The processing component can be configured to base associating the biomarker response profile with an anhedonia class on the outcome of the comparison with the predetermined classification criteria. Thereby, the biomarker response profile can be linked to a specific category increasing efficiency in generating a diagnosis for the user. The classification criteria can comprise a comparison of biomarker-based scores with normative values of these scores. The normative values can represent a non-pathological, i.e., healthy condition of the user and/or a pathological, i.e., non-healthy condition of the user. Based on the scores falling within a predetermined, preferably continuous, interval encompassing the respective normative value, the biomarker response profile can be classified by the condition represented by the respective normative value. In other words, the score can fall within a value band associated with a healthy condition or within a further value band associated with a pathological condition indicating anhedonia and/or a baseline or anticipatory arousal impairment.

The processing component can be configured to map the profile parameter to a value on an anhedonia presence or absence and/or severity scale to provide an anhedonia measure. The anhedonia measure can be separate from a classification of the biomarker response profile to a specific pathological class. For example, the anhedonia measure can be determined independent of a link to a specific normative value, value band and its assignment to a specific anhedonia class. The scale can be individualized with respect to further biomarkers of the user. These biomarkers can be used as a weight to the profile parameter such that a weighted profile parameter can be mapped to a generalized anhedonia scale. The mapping of the profile parameter can comprise applying a transfer function transforming the biomarker response profile and/or profile parameter to a capability measure that is removed from measurement specifics of the actual biomarker. As such the anhedonia measure can be compared to other anhedonia measures which may rely on different biomarkers or may be obtained by alternative systems.

The processing component can be configured to update the anhedonia measure while the reward task is provided, preferably while the task is performed by the user. This achieves the advantage that processing of the biomarker can already be executed while the task is still performed by the user. This can provide real time updates of the anhedonia measure and/or reduce processing delays once the task is complete. For example, the processing component may generate the biomarker response profile, profile parameter or anhedonia measure based on a partially complete dataset and update the respective quantity as more data is captured.

The processing component can be configured to update the biomarker response profile periodically or continuously. A continuous update can provide insights on fluctuations of the biomarker during the task. Periodical updates can increase computation efficiency and provide an increase in stability, i.e., reduction in fluctuation of values, of the output. Herein, an average can be applied to increase confidence and readability of the output, i.e., the biomarker response profile, profile parameter and/or anhedonia measure.

The processing component can be configured to update the biomarker response profile while the reward task is provided, preferably while the reward task is performed by the user.

The processing component can be configured to update the anhedonia measure periodically or continuously, in particular when a task is completed, sub-part of the task is completed or at least part of the task is completed. This can achieve the advantage that the anhedonia measure can indicate performance for part of the task. In particular a variance with respect to the type of test can be determined. The anhedonia measure may not be provided to the user during the task so as to not influence performance of the user for subsequent task.

The processing component may be configured to provide the anhedonia measure. Preferably, the anhedonia measure can be provided in relation to reference values, in particular comprising a baseline value and/or threshold values indicating a predetermined likelihood of the current anhedonia measure being a non-baseline value. This can increase efficiency and reduce the specialization required to assess the results. In particular, analysis of the biomarker response profile can be provided by the processing component to remove the need for manual mapping of the biomarker response profile or profile parameter to an anhedonia measure. The processing component can be configured to assess the anhedonia measure on a continuous scale. The scale can comprise reference values associated with specific conditions. Examples of specific conditions can comprise a healthy condition and/or pathological conditions, in particular different stages of a pathological condition representing an arousal impairment. Each condition may be associated with a mean value and/or a value band.

The processing component can be configured to provide a confidence measure corresponding to a variability of the anhedonia measure, in particular a variance and/or error measure. This can provide an estimate on the precision of the biomarker response profile, respectively profile parameter and thereto also pertaining to the anhedonia measure. An automatic classification of the biomarker response profile to an anhedonia class can be conditional on a confidence measure threshold value, i.e., the confidence may need to be high enough and/or the variance may not exceed a predetermined variance threshold value. The confidence measure may be provided in conjunction with the biomarker response profile, profile parameter and/or anhedonia measure.

The system may comprise a review component which may be configured to provide a representation of the biomarker response profile, in particular a numerical and/or graphical representation. The review component may display the biomarker response profile continuously and update a graphical representation during the performance of the task. Visualizing also the confidence measure can provide information relating to the precision of the obtained biomarker response profile already during the task. The review component may also be configured to provide a representation of the difference measure. Information provided via the review component may not be visible to the user during the task.

The review component may be configured to provide a representation of the anhedonia measure and/or the confidence measure. The review component can therefore achieve the advantage of an efficient information display based on the captured data.

The review component can be configured to provide the representation of the anhedonia measure periodically or continuously, in particular parallel to the task component providing the affective task. Thereby allowing an assessment during providing the affective task. Thereby, flexible continuation of the task can be achieved. The processing component can continue the task according to an error measure, i.e., variance, value threshold. For example, when the biomarker response profile during the task shows a high variance, the task may be continued until a lower variance is achieved. This can increase accuracy of the anhedonia measure.

The review component may be configured to provide task data to the task component. Preferably, the task component can be configured to modify the current task based on the task data. The task data can comprise a modified task and/or timing data relating to, for example, the display time of a current task, repetition of a current task and/or modified response time relating to the task. Response time can be the time interval given to the user to respond to a sub task until the next sub task is provided.

The task component may be configured to adjust a value/utility of a reinforcer of the task and/or provide a subsequent task with various reinforcer values different to the reinforcer values of the previous task. The reinforcer values can be adjusted quantitatively such that an expected relation between reinforcer values and biomarker response profile, profile parameter and/or anhedonia measure can be determined. Specifically, the processing component can correlate the reinforcer values to an expectation range for the biomarker response profile and/or profile parameter. For example, an increase in reinforcer values by a predetermined margin can determine an expected biomarker response profile, profile parameter and/or anhedonia measure. The biomarker response profile can be determined over a plurality of tasks, wherein each task corresponds to a predetermined reinforcer value. A biomarker response profile captured for a specific task may form part of a combined biomarker response profile which comprises multiple biomarker response profiles concatenated according to differences among varying reinforcer values associated with the predictive stimuli.

The system can comprise a database module configured to store a plurality of biomarker response profiles and/or anhedonia measures. The database module can serve as a resource to the processing component to determine the difference measure based on the stored plurality of biomarker response profiles and/or the stored plurality of anhedonia measures.

The processing component may be configured to compare a present biomarker response profile to the plurality of response profiles and to generate a relative measure of the biomarker response profile indicating a relation of the present biomarker response profile to the plurality of response profiles. Herein, the plurality of biomarker response profiles may form a similarity band, in particular without relying on determining a specific reference profile. In other words, the plurality of biomarker response profiles can be considered as a band of values. The band may have a varying spread in relation to the stimulus type or generally the quantity it is captured over. Herein, the processing component can be configured to assign a quality associated with the plurality of biomarker response profiles to the present biomarker response profile based on an overlap of the captured biomarker response profile with the value set spanned by the plurality of biomarker response profiles. This can reduce the processing complexity as a statistical measure may not need to be determined. The present biomarker response profile can be assessed based on similarity to profiles in the database. The processing component may provide common biomarkers of the profiles forming a similarity band to the present biomarker response profile, profile parameter and/or anhedonia measure. Thus, based on similarities of pupil responses of the user to and between the stimuli, the present biomarker response profile can be assessed. A similarity diagnostic can be provided via an interactive interface which may provide a plurality of subsets of the plurality of stored biomarker response profiles according to specific comparison parameters.

The processing component can be configured to compare a present anhedonia measure to the plurality of anhedonia measures and to generate a measure, in particular a relative measure, of the present anhedonia measure indicating a relation of the present anhedonia measure to the plurality of anhedonia measures. Thus, manual assessment of similarity can be replaced by an automatic evaluation decreasing the effort to evaluate the biomarker response profile. Herein, the database can comprise biomarker response profile associated with a specific anhedonia measure such that the processing component can compare anhedonia measures instead of biomarker response profiles.

This can increase computing efficiency since the anhedonia measures can comprise less complex data than the underlying biomarker response profiles. Furthermore, the anhedonia measure can be a means for providing diagnostic information, in particular with reduced complexity. Thereby, the anhedonia measure can be perceived as an indicator for anticipatory arousal impairment by the user directly, preferably via a corresponding smartphone app, and/or in particular with limited or without consultation of a specialist.

Additionally or alternatively, the processing component can compare biomarker response profiles, in particular on a single datapoint level which may increase computational load and/or increase precision of the comparison, i.e., a closer match to an existing biomarker response profile may be obtained. From a match or similarity to an existing biomarker response profile, respectively anhedonia measure, the processing component can infer further properties of the existing data to the presently recorded data.

The processing component can be configured to receive medical data relating to the user to which the task is provided and to provide a relevance indicator corresponding to the anhedonia measure in relation to the medical data. The medical data can correspond to a medical condition, in particular an arousal impairment and/or psychopathology such as an affective disorder, as e.g. major depressive disorder. Additionally or alternatively, the medical data can comprise further parameter data. The parameter data can relate to a quantitatively measurable biomarker. Herein, the processing component may be configured to adjust a comparison dataset from the database module based on the medical data. In particular, a comparison set corresponding to similar medical data elements can be chosen, and can in particular be used to determine the difference measure. The processing module can determine a significance of the determined anhedonia measure in relation to an existing diagnosis or a new diagnosis, preferably where the anhedonia measure indicates a quantitative measure of the severity of a medical condition.

The task component can be configured to provide a first task and a second task and wherein the second task is configured to induce a higher reinforcer value than the first task. Preferably, a set of consecutive tasks can be provided were the reinforcer value increases from task to task. Alternatively, a profile of varying reinforcer value can be provided. The task component may provide a calibration task between tasks of varying reinforcer value, wherein the calibration task may be configured to produce a baseline response of the biomarker, respectively a baseline biomarker response profile. Each task can correspond to a task condition.

The task component can be configured to provide a plurality of tasks wherein each of the tasks of the plurality of tasks has an assigned reinforcer value and wherein the task component is configured to provide the tasks based on a predetermined reinforcer value and associated anticipatory arousal profile. The reinforcer value profile may quantify the reinforcer value of each task and provide a specific order of higher reinforcer value tasks and lower reinforcer value tasks. Preferably, tasks can be provided with varying reinforcer profiles for subsequent tasks.

The task component can be configured to select reinforcer value profile from a plurality of reinforcer value profiles. The reinforcer value profile can be selected based on a profile input. Preferably, the user providing the profile input is a different user than the user performing the task such that the person performing the task is not aware of the assigned reinforcer value.

The task component can be configured to generate a task based on a task template. This achieves the advantage that individualized tasks can be provided, wherein each task may be unique or at least new to the user performing the task, i.e., the user may have processed the same type of task but not the specific task generated by the task component. A task template can define the type of task, response intervals, reinforcer value, means of providing the task, i.e., visually and/or audible.

The task component can be configured to select an affective task template from a plurality of task templates. Selecting a task template can be based on the reinforcer value profile. For example, the reinforcer value for an affective task, e.g., a reward task, to be selected can be defined, but the type of task can be arbitrary. Herein, the task component may provide a task according to stored biomarker response profiles and/or anhedonia measure to comparability of the biomarker response profile, respectively anhedonia measure which is to be captured corresponding to the generated task, respectively the selected reward task template.

The task component can be configured to select a task template and/or a task based on biomarker data and/or a biomarker response profile captured in response to a previously performed task.

The task component can be configured to select a task template and/or a task based on a predetermined expected quantitative difference in reinforcer value of the respective task template and/or task, in particular in reference to a previously and/or subsequently provided task.

The biomarker can correspond to the pupil size of the user, wherein the capture component may comprise a camera module configured to capture an image of the eye, preferably the iris, of the user and to determine the pupil size based on the captured image. The capture component and/or the processing component can be configured to track an eye-movement, in particular to generate a steady observation of the pupil diameter. The capture component can comprise a camera array, in particular at least two camera modules, configured to capture a stereoscopic image of the pupil. The camera module can be configured to remove distortions based on the projection of the three-dimensional eye onto the two-dimensional sensor plane, respectively sensor planes. The capture component may comprise a camera module for each pupil.

The capture component can comprise an illuminator configured to illuminate part of the user. Preferably the illuminator can be configured to illuminate the face and/or the eye of the user, more preferably configured to illuminate the iris and/or pupil of the user. This achieves the advantage that the pupil size can be captured with increased precision as the capture device, i.e., the camera module, may operate at a lower gain, e.g., lower ISO value, with an increased aperture value, i.e., increasing the depth of field, and/or with a decreased capture interval, i.e., shutter speed, reducing motion blur.

The illuminator can be configured to illuminate both eyes, irises and/or pupils of the user.

The illuminator can be configured to emit light in the visible spectrum and/or the infrared spectrum, preferably within the range of 700 nm to 1000 nm, preferably 850 nm to 950 nm. The capture component can be tuned to a specific spectrum, in particular corresponding to the illuminator. For example, pupillometry can be performed in the near IR-spectrum, in particular with 700 nm - 750 nm. This range can also be captured by a smartphone integrated camera, specifically a front-facing camera. Using an infrared illumination can provide increased capture precision without triggering a light induced adaption of the pupil size. The camera module may comprise an infrared image sensor configured to capture an infrared image of the eye, iris, respectively pupil.

The capture component can interface with a smartphone app allowing users to screen for anhedonia by capturing the pupil. Specifically, the camera module can use the near-infrared spectrum. Thereby, the system can provide a scalable, smartphone based assessment tool that can be used for large-scale screenings. The system can provide the basis to establish pupil response tests as minimally-invasive and inexpensive tests to aid in the detection and understanding of psychopathology such as anhedonia, as a central symptom of affective disorders such as major depressive disorder.

Using an infrared-based capture device achieves the advantage that the pupil can be easily differentiated from the iris, even in eyes with darker iris colors. Thereby, the capture component can achieve sub-millimeter accuracy across various eye colors when determining the pupil size. The capture component may be configured to simultaneously capture an infrared representation and a visible light representation of the eye, iris and/or pupil. The infrared and visual-light captures can be geometrically offset to one another. This can achieve the advantage that the stereoscopic distance between the capture device, i.e., the smartphone, and the user can be determined. The system can be configured to determine the distance between the capture component, i.e., a sensor plane of the camera and the distance to the pupil and determine the pupil size based on the distance and the pupil representation in the infrared image and/or the visual image.

By using smartphone cameras, or VR headsets, or AR glasses, users can easily check their own health, specifically independent of their location and/or without relying on expensive or specialized medical devices. This can increase the chance of detecting neuropsychiatric conditions, in particular at early stages.

The task component can implement the smartphone to visually and/or auditorily provide instructions to the user. Herein, a high ease of use based on, for example simple instructions, large fonts, clear feedback, and voice guidance can be achieved. Furthermore, the intrinsic capability of a smart device to share information can be used to easily provide feedback and/or results to medical personnel.

The capture component can be configured to continuously determine the biomarker with a sampling rate greater or equal to a predetermined threshold rate of 1 Hz, 10 Hz, 25 Hz, 50 Hz or 100 Hz. Preferably, the capture rate is at least an order of magnitude higher than the average rate of change of the biomarker in response to the task. The sampling rate can be adjusted based on a selected biomarker, i.e., according to a maximum speed of the biomarker changing its value, in particular a value change above the minimum resolution of the capture component. Consequently, the capture component can be configured to capture the biomarker at a sampling rate that corresponds to the maximum rate of change of the biomarker such that two captures of the biomarker are within two resolution steps of the capture component. For example, the capture rate of a camera is high enough such that a spatial change of the biomarker, i.e. the size of the pupil between two captures does not exceed two pixels. For example, the capture rate and/or the resolution of the capture component, e.g. an image sensor, is chosen in such a fashion that capturing the movement and/or dilation of the pupil can be performed at the maximally achievable spatial resolution. A boundary between the iris and pupil may progress only by 1 pixel between two captured frames. Alternatively, the resolution can be set to be coarser, such that a shift of the boundary between iris and pupil of 2 pixels or more can be registered. Alternatively, the resolution of the capture component can be specified by a spatial resolution in millimeters. For example, the capture component can be configured to capture the pupil dilation with a precision of 0.5 mm, preferably 0.2 mm, more preferably 0.1 mm in reference to a pupil diameter.

The capture component can be configured to determine a relative value change of the biomarker.

The capture component can comprise a sensor, in particular an image sensor, configured to capture the biomarker data. The processing component can be configured to determine the biomarker within a tolerance of up to 5%, preferably up to 1%, more preferably only up to 0.1%. The precision of the biomarker capture can also be determined by the accuracy of capture component when capturing, i.e., measuring the biomarker. Tolerance can be defined as an allowable deviation from a standard or a specification. Accuracy can be defined as the closeness of a measurement to the true value. The processing component can be configured to determine a correlation measure representing a consistency of the captured biomarker data, respectively biomarker response profile. Preferably the system can be configured to determine a Pearson Correlation. Herein, the system can be configured to achieve a Pearson Correlation Coefficient above 0.7, preferably above 0.80. A split-half reliability measure can also be used to determine the level of correlation.

A high precision in determining the biomarker can translate to a high accuracy in determining the anhedonia measure or at least translate to a higher confidence of the biomarker response profile such that a distinction between an inconspicuous profile or a pathological profile can be made with increased certainty, reducing the risk of false positives and/or false negatives. The camera module can comprise the sensor.

The capture component can be configured to capture the biomarker during a time interval associated with the reward task. Thereby, the biomarker data can be directly related to the corresponding task.

The capture module can be configured to determine the biomarker for a first time interval prior to the task being provided to the user, for a second time interval during which the task is provided to the user and/or for a third time interval after the task is no longer provided to the user. This can achieve the advantage that a hysteresis of the biomarker can be captured. Hysteresis in the biomarker response profile may occur when the biomarker not only on the current task, but also on a past task. Thereto, the biomarker may show a memory effect based on its previous states and may not respond immediately or linearly to changes in the input, i.e. a change in reinforcer value and associated expectancy for the respective predictive stimulus. In particular, the system can determine whether the biomarker returns to a predetermined baseline level between tasks and/or whether the tasks have a compounding component, i.e., the biomarker stays at the end value captured at the tasks completion or does adjust to an intermediate value between a baseline level and a further level captured during the task. Additionally, and/or alternatively, the biomarker value of a subsequent task can be impacted by a previous task. Any biomarker capture of these intervals can form part of the biomarker response profile. A sequence of time intervals can comprise a first interval, a plurality of subsequent second intervals, where each second interval is associated with a corresponding task and/or a third time interval. Alternatively, a time interval after the task can also form a prior time interval for a subsequent task.

The sensor can be configured to track a pupil size of the user. Additionally or alternatively, the processing component and/or the capture component can be configured to track the pupil size based on the sensor input, i.e., the sensor captures a spatial range comprising all possible positions of the pupil and the processing component, respectively the capture component can be configured to track the pupil based on the sensor data.

The sensor can be configured to track an eye movement of the user. Herein the sensor and/or the capture component can account for eye movement and adjust accordingly. This can achieve the advantage that the data provided by the capture component, respectively the biomarker data, can comprise a steady representation of the biomarker increasing the efficiency of processing the biomarker data.

The processing component can be configured to determine a focus area based on the eye movement, preferably wherein the focus area is a subsection of a display area through which the task is provided.

The system can comprise a display component configured to display the task to the user. The display component can also be configured to display data provided by the review component, in particular a representation of the biomarker response profile, the profile parameter, the difference measure and/or the anhedonia severity measure. The display component can be scaled according to the distance to the user such that a predetermined minimum area of the field of vision of the user is covered by the display component. The size of the display component can be scaled according to the task. This can achieve the advantage that the task can be displayed at an optimal scaling such that the visual representation of the task may not distract from the task itself.

The system can comprise a headset component configured to mount onto the head of a user and to position the display component at least partially in the field of vision of the user, preferably to dispose a primary area defined by the task within the field of vision of the user. The headset component can be a virtual reality headset or an augmented reality headset. Alternatively, the display component can be a smartphone and the headset component can be configured to hold the smartphone in the field of vision of the user. This can achieve the advantage that the display component can take up a majority of the field of vision of the user thereby reducing the influence of distractions and increasing focus on the task. Thereby, variance of the correlation of the biomarker and the task and the anhedonia measure and/or anticipatory arousal can be decreased. The combination of a smartphone and the headset component can achieve a constant scaling of the displayed task such that comparability of a plurality of biomarker response profiles captured for a plurality of users can be increased.

The headset component can be configured to at least partially block ambient light from entering the field of vision of the user. This can increase the overall focus of the user onto the task. Ambient distractions may not be visible to the user. Additionally or alternatively, the headset component may be configured to block ambient sounds to minimize distractions. The processing component may be configured to determine an influence of ambient parameters, i.e., ambient light, ambient sound and/or any ambient parameters (pressure, temperature, humidity), on the user and adjust the task accordingly. For example, an increase in variance of the biomarker may indicate an influence of ambient parameters. The task can be halted, repeated or aborted based on the variance of the biomarker exceeding a threshold value. Additionally or alternatively, ambient threshold values can be defined, wherein the processing component may provide task data to the task component to adjust the task accordingly. The headset component may be configured to shield the user from any visual and audible input besides the task.

The display component can comprise two display units configured to display the task in conjunction with one another, where each of the display units is associated with one eye of the user. The system can be configured to process biomarker data relating to a single eye or configured to process biomarker data relating to both eyes. The number of eyes to be tracked can be determined by the type of neuropsychiatric or neurological disease to be investigated.

The system can comprise a calibration component configured to determine reinforcer value and the expectancy of a predictive stimulus of a provided reward task. The calibration component can thus determine reinforcer value scaling of tasks relative to one another. Additionally or alternatively the calibration component may determine an absolute scaling of stimuli relative to one another. Herein, a first baseline level and/or initial value of the biomarker response profile can be determined. Thereto reinforcer value of the task can be determined by averaging over a plurality of individuals of a control group. The calibration can be adjusted when further users fitting the control group perform the task. This may increase the overall precision of the reinforcer value assigned to a specific task. The calibration can be performed as an initialization step of the system. The calibration component may also be configured to determine reference values with respect to the present user based on datasets stored in the database module. Thereby, the calibration component may individualize a reference profile, in particular according to parameters of the user which preferably associate the user with a group of users that can be characterized by similar biomarker response profiles. As such an initial calibration can increase the precision of a classification based on the biomarker response profile and/or increase the precision of the anhedonia measure.

The calibration component can be configured to determine a baseline level of the biomarker associated with a baseline reinforcer value level.

The calibration component can be configured to determine a maximum level of the biomarker associated with a maximum reinforcer value level. The calibration component can thereby determine a maximum range and/or an absolute range for the biomarker. The calibration component can be configured to weigh a biomarker response profile according to a determined baseline level and/or maximum level of the biomarker. This can increase comparability of the biomarker response profile to previously captured biomarker response profiles.

The calibration component can be configured to determine at least one intermediate level of the biomarker associated with a corresponding intermediate reinforcer value level. Thereby an individualized scaling of the biomarker in reference to reinforcer values can be determined. For example, the biomarker may scale linearly or non-linearly with the reinforcer values and/or expectancy of the predictive stimuli imposed by the task. The calibration component may increase the number of reference values to increase the precision in determining the scaling of the biomarker in reference to the stimulus categories.

The calibration component can be configured to generate, preferably interpolate and/or extrapolate, various categories of the stimuli, in particular wherein each category, and the difference among categories, corresponds a representative biomarker value. The scale can be a quantitative scale such that the reinforcer value can be increased and/or decreased by a specific value. Alternatively, the scale can be ordinal such that the reinforcer value increases or decreases and the scaling may be non-linear, and possibly even categorical. The processing component can be configured to determine a statistical relationship between the biomarker, e.g., the pupil size, and the stimulus categories, in particular when the stimulus categories are considered ordinal/nominal.

The task component can be configured to provide a set of tasks, wherein reinforcer value of subsequent stimuli differs based on a qualitative or quantitative scale of the reinforcer value. The difference may be quantified by a predetermined value. Alternatively, an increase or a decrease of reinforcer value for the subsequent task can be defined based on the reinforcer value scale.

The task component can provide a task comprising task conditions that differ in a pseudo-random fashion. For example, the task conditions can be provided in an interspersed manner, e.g., stimulus 1, stimulus 3, stimulus 2, stimulus 3, stimulus 2, stimulus 1, etc. The task component can be configured to pseudo-randomize stimuli provided to the user. This can increase task engagement by the user.

According to a second aspect the invention relates a method for assessing anhedonia, comprising the steps of providing a task, in particular a reward task to a user, capturing biomarker data of a biomarker of the user in response to the task and generating a biomarker response profile based on the biomarker data.

The method can comprise the step of determining a profile parameter based on the biomarker response profile and assessing anhedonia, preferably the presence or absence and/or the severity of anhedonia, of the user based on the profile parameter.

The method can further comprise the step of providing a stimulus perceivable by the user.

The method can comprise the step of providing the stimulus for a predetermined time interval. The stimulus can have a duration of at least 1 second, preferably a duration of 4 to 8 seconds.

The method can comprise the step of providing a plurality of stimuli, preferably providing a set of stimuli comprising up to 15 stimuli.

The method can comprise the step of providing a trigger signal. Herein, the trigger signal can differ substantially in perception by the user with respect to the stimulus.

The method can comprise the step of providing the trigger signal after the stimulus, preferably directly after the stimulus and/or after a brief pause.

The method can comprise the step of capturing the stimulus response and/or initiating the stimulus response by the trigger signal being provided.

The method can comprise the step of capturing a stimulus response for each provided stimulus.

The method can comprise the step of assessing the stimulus response based on the provided stimulus according to a set of task rules.

The method can comprise the step of determining a response time, preferably a time interval between the stimulus and the stimulus response by the user.

The method can comprise the step of matching the stimulus response to the stimulus based on the time interval.

The method can comprise the step of providing a reward to the user based on a reward condition being met.

The method can comprise the step of providing a reward associated with a primary reinforcer.

The method can comprise the step of providing a reward associated with a secondary reinforcer.

The method can comprise the step of providing at least one of the following rewards: a primary reward; and/or a secondary reward, such as a symbol or image, a monetary reward, preferably an auditory and/or visual confirmation of the acquisition of the monetary reward by the user, and a predetermined number of points.

The method can comprise the step of providing the reward condition as part of the task to the user, preferably prior to the task being provided.

The method can comprise the step of applying a predetermined control reward condition when processing the captured stimulus response, preferably wherein the predetermined control reward condition comprises a response time captured below a maximum response time threshold value.

The method can comprise the step of providing the control trigger within a predetermined time interval after the control stimulus.

The method can comprise the step of providing an identical signal as the response trigger and the control trigger.

The method can comprise the step of providing a first stimulus as the reward stimulus and a further stimulus as the control stimulus. The first stimulus and the further stimulus can be distinguishable with respect to one another by the user.

The method can comprise the step of spatially filtering the reward stimulus, the control stimulus and/or the passive stimulus.

The method can comprise the step of determining and/or removing effects of the reward stimulus and/or the control stimulus presentation from the biomarker data, preferably removing signal cross-contamination from the stimulus presentation into the biomarker data.

The method can comprise the step of providing a segmented task comprising at least one of: an anticipation phase, which preferably comprises the reward stimulus, the control stimulus and/or the passive stimulus; a response trigger phase; a response capture phase; a feedback phase; a reward consumption phase; and/or a fixation phase.

The method can comprise the step of capturing biomarker data during at least one phase of the segmented task, preferably during each phase of the segmented task.

The method can comprise the step of synchronizing capturing the biomarker data with the reward stimulus, preferably such that a time interval before, during and/or after the reward stimulus is captured.

The method can comprise the step of synchronizing capturing the biomarker data with the control stimulus, preferably such that a time interval before, during the secondary control stimulus is captured.

The method can comprise the step of determining a derivative of the biomarker vector, preferably a first derivative and/or a second derivative.

The method can comprise the step of applying a fit to at least part of the biomarker vector, in particular a linear or non-linear fit.

The method can comprise the step of segmenting the biomarker vector and applying a fit to at least one segment of the biomarker vector, preferably applying differing fits to different segments of the biomarker vector.

The method can comprise the step of determining a profile parameter based on the fit, preferably determining a fit characteristic, e.g., an intercept value and/or an acceleration value, of the biomarker vector.

The method can comprise the step of applying the fit time-bin-wise to the biomarker vector, preferably wherein each bin represents a predetermined time interval, in particular a time-interval between 0.5-2.0 s.

The method can comprise the step of repeating a task for a plurality of instances.

The method can comprise the step of comparing stimuli responses based on the plurality of captured biomarker responses corresponding to the respective task instances.

The method can comprise the step of wherein the processing component is configured to determine the profile parameter for each task instance and to compare the profile parameter across the task instances.

The method can comprise the step of determining an anhedonia measure and/or anticipatory arousal level, in particular by matching the biomarker profile parameter to the stimulus types, in particular with a quantitative or qualitative scale.

The method can comprise the step of repeating a task or a task component for a predetermined number of times.

The method can comprise the step of providing the repeated tasks or task components in a predetermined sequence, in particular in a predetermined sequence of stimuli, preferably repeating stimuli.

The method can comprise the step of training a machine learning model based on the biomarker training data. The machine learning model can be adapted to identify signal features that characterize anhedonia in the user.

The method can comprise the step of applying the machine learning model to biomarker data and to output of the presence or absence and/or severity of anhedonia of the user based on the signal features

The method can comprise providing task data to the task component, modifying the current reward task based on the task data and/or performing a cross-validation on the trained machine learning model using a validation set of biomarker data. The cross-validation can comprise evaluating the performance of the machine learning model on the validation set and adjusting one or more parameters of the machine learning model based on the evaluation.

The method can comprise the step of performing a feature extraction on the biomarker data. The feature extraction can comprise applying one or more filters and/or transformations to the biomarker data to obtain a reduced set of features that are relevant for anhedonia identification.

The method can comprise the step of applying the machine learning model to the preprocessed biomarker data and to output a level of anhedonia in the subject based on the signal features.

The method can comprise the step of determining a difference measure and to assess the presence or absence and/or severity of anhedonia of the user based on the difference measure.

The method can comprise the step of determining an anhedonia measure based on the biomarker response profile and/or the difference measure

The method can comprise the step of comparing the difference measure and/or the biomarker response profile against a set of predetermined values and associating the biomarker response profile with an anhedonia class, which in particular corresponds to a pathological anhedonia of the user.

The method can comprise the step of mapping the profile parameter to a value on an anhedonia scale to provide an anhedonia measure.

The method can comprise the step of updating the anhedonia measure while the task is provided, preferably while the reward task is performed by the user.

The method can comprise the step of updating the biomarker response profile periodically or continuously.

The method can comprise the step of update the biomarker response profile while the reward task is provided, preferably while the reward task is performed by the user.

The method can comprise the step of updating the anhedonia measure periodically or continuously, in particular when a task is completed, a sub-part of the task is completed or at least part of the task is completed.

The method can comprise the step of providing the anhedonia measure, in particular to a review component.

The method can comprise the step of providing a confidence measure corresponding to a variability of the anhedonia measure, in particular a variance and/or error measure.

The method can comprise the step of providing a representation of the biomarker response profile, in particular a numerical and/or graphical representation.

The method can comprise the step of providing a representation of the anhedonia measure and/or the confidence measure.

The method can comprise the step of providing the representation of the anhedonia measure periodically or continuously, in particular parallel to the task component providing the reward task.

The method can comprise the step of providing task data to the task component and wherein the task component is configured to modify the current reward task based on the task data.

The method can comprise the steps of adjusting a reinforcer value of a given stimulus type of the reward task and/or providing a subsequent task with a reinforcer value different to the reinforcer value of the previous task.

The method can comprise the step of storing a plurality of biomarker response profiles and/or anhedonia measures.

The method can comprise the steps of comparing a present biomarker response profile to the plurality of response profiles and/or generating a relative measure of the biomarker response profile indicating a relation of the present biomarker response profile to the plurality of response profiles.

The method can comprise the steps of comparing a present anhedonia measure to the plurality of anhedonia measures and generating a measure, in particular a relative measure, of the present anhedonia measure indicating a relation of the present anhedonia measure to the plurality of anhedonia severity measures

The method can comprise the steps of receiving medical data relating to the user to which the task is provided and providing a relevance indicator corresponding to the anhedonia measure in relation to the medical data.

The method can comprise the step of providing a plurality of tasks wherein each of the tasks of the plurality of tasks has a predetermined reinforcer value. The tasks can be provided based on a predetermined stimulus type profile.

The method can comprise the step of selecting a stimulus type profile from a plurality of stimulus type profiles.

The method can comprise the step of generating a task based on a reward task template.

The method can comprise the step of selecting a reward task template from a plurality of task templates.

The method can comprise the step of selecting a task template and/or a task based on biomarker data and/or a biomarker response profile captured in response to a previously performed task.

The method can comprise the step of selecting a task template and/or a task based on a predetermined expected quantitative difference in reinforcer value of the respective stimulus type of the respective task template and/or task, in particular in reference to a previously and/or subsequently provided task.

The method can comprise the step of providing a fixation task, in particular as part of the plurality of tasks.

The biomarker can correspond to a pupil size of the user. The method can comprise the steps of capturing an image of the eye of the user, preferably the iris of the user, and determining the pupil size based on the captured image.

The method can comprise the step of illuminating part of the user, preferably illuminating the face and/or the eye of the user, more preferably illuminating the iris and/or pupil of the user.

The method can comprise the step of illuminate both eyes, irises and/or pupils of the user.

The method can comprise the step of emitting light in the visible spectrum and/or the infrared spectrum, preferably within the range of 700 nm to 1000 nm.

The method can comprise the step of continuously determining the biomarker with a sampling rate greater or equal to a predetermined threshold rate of 1 Hz, 10 Hz, 25 Hz, 50 Hz or 100 Hz.

The method can comprise the step of determining a relative value change of the biomarker.

The method can comprise the step of capturing the biomarker data, in particular determining the biomarker with a relative tolerance of up to 5%, preferably up to 1%, more preferably up to 0.1% based on the biomarker data.

The method can comprise the step of capturing the biomarker during a time interval associated with the reward task.

The method can comprise the step of determining the biomarker for a first time interval prior to the task being provided to the user, for a second time interval during which the task is provided to the user and/or for a third time interval after the reward task is no longer provided to the user.

The method can comprise the step of tracking a pupil size of the user.

The method can comprise the step of tracking an eye movement of the user.

The method can comprise the step of determining a focus area based on the eye movement, preferably determining if the focus area coincides with a display area providing the task or at least partially coincides with the display area.

The method can comprise the step of displaying the task to the user.

The method can comprise the step of mounting a headset component onto the head of a user and disposing the display component at least partially in the field of vision of the user, preferably disposing a primary area defined by the task within the field of vision of the user.

The method can comprise the step of at least partially blocking ambient light from entering the field of vision of the user.

The method can comprise the step of displaying the task by means of two display units in conjunction with one another. Each of the display units can be associated with one eye of the user.

The method can comprise the step of determining an anhedonia measure and/or anticipatory arousal level of a provided reward task.

The method can comprise the step of determining a baseline level of the biomarker associated with a baseline level.

The method can comprise the step of determine a maximum level of the biomarker associated with a maximum response.

The method can comprise the step of determining at least one intermediate level of the biomarker associated with a corresponding intermediate response.

The method can comprise the step of generating, in particular interpolating and/or extrapolating, a variety of reinforcer values. The stimulus type can be predictive of a certain reinforcer value corresponding to a representative biomarker value, and/or profile parameter.

The method can comprise the step of providing a set of tasks, wherein the reinforcer value of subsequent tasks is predetermined to increase or to decrease, preferably by a predetermined value, based on the scale and/or category of reinforcer value levels.

The method can comprise the step of determining the profile parameter based on the difference in stimulus type in subsequent tasks.

Below, system embodiments will be discussed. These embodiments are abbreviated by the letter "S" followed by a number. Whenever reference is herein made to "system embodiments", these embodiments are meant.
S1. System (100) for assessing anhedonia, comprising
   a task component (101) configured to provide a task, in particular a reward task to a user;
   a capture component (102) configured to capture biomarker data of a biomarker of the user in response to the task;
   a processing component (103) configured to generate a biomarker response profile based on the biomarker data.
S2. System according to the preceding embodiment, wherein the processing component is configured to determine a profile parameter based on the biomarker response profile and to assess anhedonia, preferably the presence or absence and/or the severity of anhedonia, basic arousal and/or anticipatory arousal of the user based on the profile parameter.
S3. System according to any of the preceding embodiments, wherein the task component comprises a presentation module configured to provide a stimulus perceivable by the user.
S4. System according to any of the preceding embodiments, with features of S3, wherein the stimulus comprises at least one of a geometric pattern, an image, preferably an image of an object, a symbol, and/or an auditory stimulus.
S5. System according to any of the preceding embodiments, with features of S3, wherein the task component is configured to provide the stimulus for a predetermined time interval, in particular wherein the stimulus has a duration of at least 1 second, preferably a duration of 4 to 8 seconds.
S6. System according to any of the preceding embodiments, with features of S3, wherein the task component is configured to provide a plurality of stimuli, preferably to provide a set of stimuli comprising up to 15 stimuli.
S7. System according to any of the preceding embodiments, with features of S6, wherein each stimulus of the plurality of stimuli corresponds to a predetermined reinforcer value, preferably wherein the reinforcer values of the stimuli differ substantially.
S8. System according to any of the preceding embodiments, with features of S6, wherein the reinforcer value and/or the expectancy value of the predictive stimulus varies according to stimulus category and the eventual biomarker response of the user.
S9. System according to any of the preceding embodiments, wherein the task component is configured to provide a trigger signal, in particular wherein the trigger signal differs substantially in perception by the user with respect to the stimulus.
S10. System according to any of the preceding embodiments, with features of S9, wherein the task component is configured to provide the trigger signal after the stimulus, preferably directly after the stimulus or after a pause.
S11. System according to any of the preceding embodiments, with features of S9, wherein the trigger signal or the end of the trigger signal defines the start of a response interval in which a stimulus response by the user is captured.
S12. System according to any of the preceding embodiments, with features of S9, wherein the trigger signal comprises a visual and/or auditory signal, in particular a light flash.
S13. System according to any of the preceding embodiments, with features of S3, wherein the capture component is configured to capture the stimulus response and/or wherein the stimulus response is initiated by the trigger signal being provided.
S14. System according to any of the preceding embodiments, with features of S13, wherein the stimulus response comprises at least one of
   a motor response;
   an auditory, respectively verbal response;
   an expression response; and/or
   a neural response.
S15. System according to any of the preceding embodiments, with features of S6, wherein the capture component is configured to capture a stimulus response for each provided stimulus.
S16. System according to any of the preceding embodiments, with features of S15, wherein the processing component is configured to assess the stimulus response based on the provided stimulus according to a set of task rules.
S17. System according to any of the preceding embodiments, with features of S16, wherein assessing the stimulus response comprises matching the stimulus response to the stimulus.
S18. System according to any of the preceding embodiments, with features of S16, wherein the processing module is configured to determine a response time, preferably a time interval between the stimulus and the stimulus response by the user.
S19. System according to any of the preceding embodiments, with features of S18, wherein, the processing component is configured to match the stimulus response to the stimulus based on the time interval.
S20. System according to any of the preceding embodiments, comprising a reward module configured to provide a reward to the user based on a reward condition being met.
S21. System according to any of the preceding embodiments, with features of S20, wherein the reward module is configured to provide a reward associated with a primary reinforcer.
S22. System according to any of the preceding embodiments, with features of S20, wherein the reward module is configured to provide a reward associated with a secondary reinforcer.
S23. System according to any of the preceding embodiments, with features of S20, wherein the reward condition comprises at least one of
   the time interval not exceeding a maximum threshold value;
   the stimulus response corresponding to an expected stimulus response type
   a stimulus response pattern corresponding to an expected stimulus pattern (e.g., a similarity to norm response);
   the stimulus response adhering to the task rules;
S24. System according to any of the preceding embodiments, with features of S20, wherein the reward module is configured to provide at least one of the following rewards:
   - a primary reward;
   - a secondary reward, such as a symbol or image, a monetary reward, preferably an auditory and/or visual confirmation of the acquisition of the monetary reward by the user, and a predetermined number of points.
S25. System according to any of the preceding embodiments, with features of S20, wherein the task component is configured to provide the reward condition as part of the task to the user, preferably prior to the task being provided.
S26. System according to any of the preceding embodiments, wherein the task comprises a reward sequence for capturing biomarker data in relation to an anticipated reward and/or a control sequence for capturing biomarker data in relation to anticipating a different reward, which in particular is of a different value to the user, and/or a passive sequence for capturing biomarker data in relation to the lack of a trigger and/or reward.
S27. System according to any of the preceding embodiments, wherein the reward sequence comprises a reward stimulus, wherein the reward stimulus indicates the potential for receiving a reward to the user.
S28. System according to any of the preceding embodiments, with features of S27 wherein the reward sequence comprises a reward trigger at a predetermined time interval after the reward stimulus.
S29. System according to any of the preceding embodiments, with features of S27 wherein the task component is configured to provide the reward trigger within a predetermined time interval after the reward stimulus.
S30. System according to any of the preceding embodiments, with features of S27, wherein the processing component is configured to apply a predetermined control reward condition when processing the captured stimulus response, preferably wherein the predetermined control reward condition comprises a response time captured below a maximum response time threshold value.
S31. System according to any of the preceding embodiments, wherein the control sequence comprises a control stimulus, and wherein the control stimulus indicates that there is the potential for the user to receive a reward of lesser value, e.g. positive verbal feedback, a checkbox or other image.
S32. System according to any of the preceding embodiments, with features of S29 and S31, wherein the control sequence comprises a control trigger at a predetermined time after the control stimulus.
S33. System according to any of the preceding embodiments, with features of S31, wherein the task component is configured to provide the control trigger within a predetermined time interval after the control stimulus.
S34. System according to any of the preceding embodiments, with features of S31, wherein the passive sequence comprises a passive stimulus, wherein the passive stimulus indicates the absence of a trigger signal and that there is no potential for the user to receive a reward.
S35. System according to any of the preceding embodiments, with features of S31, wherein the passive sequence comprises a trigger signal, in particular wherein the trigger signal is provided after the passive stimulus, and wherein the passive stimulus indicates that there is no potential for the user to receive a reward.
S36. System according to any of the preceding embodiments, with features of S27 and S31 wherein the task component is configured to provide an identical signal as the response trigger and the control trigger.
S37. System according to any of the preceding embodiments, with features of S27 and S31, wherein the task component is configured to provide a first stimulus as the reward stimulus and a further stimulus as the control stimulus, and wherein the first stimulus and the further stimulus are distinguishable with respect to one another by the user.
S38. System according to any of the preceding embodiments, wherein the reward stimulus, the control stimulus and/or the passive stimulus are impact neutral with respect to the biomarker data, biomarker response profile and/or profile parameter when being perceived by the user without reward context.
S39. System according to any of the preceding embodiments, wherein the reward stimulus, the control stimulus and/or the passive stimulus can be geometric transformations with respect to one another, in particular when being perceived by the user without reward context.
S40. System according to any of the preceding embodiments, wherein the reward stimulus, the control stimulus and/or the passive stimulus are defined within a predetermined biomarker response value interval.
S41. System according to any of the preceding embodiments, wherein the capture component and/or the processing component are configured to spatially filter the reward stimulus, the control stimulus and/or the passive stimulus.
S42. System according to any of the preceding embodiments, wherein the capture component is configured to determine and/or to remove effects of the reward stimulus presentation and/or the control stimulus presentation from the biomarker data, preferably to remove signal cross-contamination from the stimulus into the biomarker data.
S43. System according to any of the preceding embodiments, wherein the task component is configured to provide a segmented task comprising at least one of:
   an anticipation phase, which preferably comprises the reward stimulus, the control stimulus and/or the passive stimulus;
   a response trigger phase;
   a response capture phase;
   a feedback phase;
   a reward consumption phase; and/or
   a fixation phase.
S44. System according to any of the preceding embodiments, with features of S43, wherein the capture component is configured to capture biomarker data during at least one phase of the segmented task, preferably during each phase of the segmented task.
S45. System according to any of the preceding embodiments, wherein the capture component is configured to synchronize capturing the biomarker data with the reward stimulus, preferably such that a time interval before, during and/or after the reward stimulus is captured.
S46. System according to any of the preceding embodiments, wherein the capture component is configured to synchronize capturing the biomarker data with the control stimulus, preferably such that a time interval before, during and/or after the secondary control stimulus is captured.
S47. System according to any of the preceding embodiments, wherein the biomarker data comprises a biomarker vector, preferably a pupil size vector.
S48. System according to any of the preceding embodiments, with features of S47, wherein the processing component is configured to determine a derivative of the biomarker vector, preferably a first derivative and/or a second derivative.
S49. System according to any of the preceding embodiments, wherein the processing component is configured to apply a fit to at least part of the biomarker vector, in particular a linear or non-linear fit.
S50. System according to any of the preceding embodiments, with features of S49, wherein the processing component is configured to segment the biomarker vector and to apply a fit to at least one segment of the biomarker vector, preferably wherein the processing component is configured to apply differing fits to different segments of the biomarker vector.
S51. System according to any of the preceding embodiments, with features of S49, wherein the processing component is configured to determine a profile parameter based on the fit, preferably to determine a fit characteristic, e.g., an intercept value and/or an acceleration value, of the biomarker vector.
S52. System according to any of the preceding embodiments, with features of S49, wherein the processing component is configured to apply the fit time-bin-wise to the biomarker vector, preferably wherein each bin represents a predetermined time interval, in particular a time-interval between 0.5-2.0 s.
S53. System according to any of the preceding embodiments, with features of S52, wherein the segment corresponds to at least one of the following:
   a first time interval starting with the reward stimulus or the control stimulus, preferably wherein the first time interval is binned, in particular binned in 1 s intervals;
   an onset time interval starting at a predetermined time, preferably between 100 ms and 2000 ms, more preferably 500 ms or 1000 ms before the reward trigger, respectively the control trigger;
   a finalizing time interval ending with the subsequent stimulus, preferably starting 500 ms and 5000 ms, more preferably 2000 ms before the subsequent stimulus;
   a predetermined segment between the stimulus and the subsequent stimulus; the full stimulus duration.
S54. System according to any of the preceding embodiments, wherein the task component is configured to repeat a task for a plurality of instances.
S55. System according to any of the preceding embodiments, with features of S54, wherein the processing component is configured to compare stimuli responses based on the plurality of captured biomarker responses corresponding to the respective task instances.
S56. System according to any of the preceding embodiments, with features of S54, wherein the processing component is configured to determine the profile parameter for each task instance and to compare the profile parameter across the task instances.
S57. System according to any of the preceding embodiments, with features of S56, wherein the processing component is configured to determine an anhedonia measure and/or anticipatory arousal level , preferably based on the comparison outcome of the profile parameters, in particular by matching the biomarker profile parameter to the stimulus types, in particular with a quantitative or qualitative scale.
S58. System according to any of the preceding embodiments, wherein the task component is configured to repeat a task or a task component for a predetermined number of times.
S59. System according to any of the preceding embodiments, with features of wherein the task component is configured to provide the repeated tasks or task components in a predetermined sequence, in particular in a predetermined sequence of stimuli, preferably repeating stimuli.
S60. System according to any of the preceding embodiments, comprising a machine learning component configured to train a machine learning model based on biomarker training data, wherein the machine learning model is adapted to identify signal features that characterize anhedonia in the user.
S61. System according to any of the preceding embodiments, with features of S60, wherein the processing component is configured to apply the machine learning model to biomarker data and to output of the presence or absence and/or severity of anhedonia of the user based on the signal features.
S62. System according to any of the preceding embodiments, with features of S60, wherein the machine learning model is a neural network that comprises one or more hidden layers and an output layer, wherein the output layer provides a probability distribution over different levels of anhedonia.
S63. System according to any of the preceding embodiments, with features of S60, wherein the machine learning component is configured to perform a cross-validation on the trained machine learning model using a validation set of biomarker data, wherein the cross-validation comprises evaluating the performance of the machine learning model on the validation set and adjusting one or more parameters of the machine learning model based on the evaluation.
S64. System according to any of the preceding embodiments, with features of S60, wherein the processing component is configured to perform a feature extraction on the biomarker data, wherein the feature extraction comprises applying one or more filters and/or transformations to the biomarker data to obtain a reduced set of features that are relevant for anhedonia identification.
S65. System according to any of the preceding embodiments, with features of S61, wherein the processing component is configured to apply the machine learning model to the preprocessed biomarker data and to output a level of anhedonia in the subject based on the signal features.
S66. System according to any of the preceding embodiments, wherein the profile parameter corresponds to a statistical measure and/or a parameter of a mathematical function, in particular a function fitted to the biomarker response profile.
S67. System according to any of the preceding embodiments with features of S2, wherein the processing component is configured to determine a difference measure and to assess the presence or absence and/or severity of anhedonia of the user based on the difference measure.
S68. System according to the preceding embodiment, wherein the difference measure corresponds to at least one of the following:
   difference of the profile parameter to a predetermined baseline value;
   difference of a fitted curve parameter to a predetermined fitted curve parameter;
   similarity of the biomarker response profile to a reference profile;
   scaling of the biomarker response profile in comparison to a reference profile;
   scaling of a parameter fitted to the biomarker response profile in comparison to a reference parameter.
S69. System according to any of the preceding embodiments comprising features of S66, wherein the processing component is configured to determine an anhedonia measure based on the biomarker response profile and/or the difference measure.
S70. System according to any of the preceding embodiment, wherein the processing component is configured to compare the difference measure and/or the biomarker response profile against a set of predetermined values and to associate the biomarker response profile with an anhedonia class, which in particular corresponds to a pathological anhedonia of the user.
S71. System according to any of the preceding embodiments, wherein the processing component is configured to map the profile parameter to a value on an anhedonia scale to provide an anhedonia measure.
S72. System according to any of the preceding embodiment, wherein the processing component is configured to update the anhedonia measure while the cognitive task is provided, preferably while the reward task is performed by the user.
S73. System according to any of the preceding embodiments, wherein the processing component is configured to update the biomarker response profile periodically or continuously.
S74. System according to the preceding embodiment, wherein the processing component is configured to update the biomarker response profile while the reward task is provided, preferably while the reward task is performed by the user.
S75. System according to any of the preceding embodiments comprising features of S69, wherein the processing component is configured to update the anhedonia measure periodically or continuously, in particular when a task is completed, sub-part of the task is completed or at least part of the task is completed.
S76. System according to the preceding embodiment, wherein the processing component is configured to provide the anhedonia measure, in particular to a review component.
S77. System according to any of the preceding embodiments, wherein the processing component is configured to provide a confidence measure corresponding to a variability of the anhedonia measure, in particular a variance and/or error measure.
S78. System according to any of the preceding embodiments, comprising the review component (106) configured to provide a representation of the biomarker response profile, in particular a numerical and/or graphical representation.
S79. System according to the preceding embodiment, wherein the review component is configured to provide a representation of the anhedonia measure and/or the confidence measure.
S80. System according to the preceding embodiment, wherein the review component is configured to provide the representation of the anhedonia measure periodically or continuously, in particular parallel to the task component providing the reward task.
S81. System according to any of the preceding embodiments comprising features of S78, wherein the review component is configured to provide task data to the task component and wherein the task component is configured to modify the current reward task based on the task data.
S82. System according to any of the preceding embodiments comprising features of S78, wherein the task component is configured to adjust reinforcer value of a given stimulus type of the reward task and/or provide a subsequent task with a reinforcer value different to the reinforcer value of the previous task.
S83. System according to any of the preceding embodiments, comprising a database module (105) configured to store a plurality of biomarker response profiles and/or anhedonia measures.
S84. System according to the preceding embodiment, wherein the processing component is configured to compare a present biomarker response profile to the plurality of response profiles and to generate a relative measure of the biomarker response profile indicating a relation of the present biomarker response profile to the plurality of response profiles.
S85. System according to any of the preceding embodiments comprising features of S83, wherein the processing component is configured to compare a present anhedonia measure to the plurality of anhedonia measures and to generate a measure, in particular a relative measure, of the present anhedonia measure indicating a relation of the present anhedonia measure to the plurality of anhedonia severity measures.
S86. System according to any of the preceding embodiments, wherein the processing component is configured to receive medical data relating to the user to which the task is provided and to provide a relevance indicator corresponding to the anhedonia measure in relation to the medical data.
S87. System according to any of the preceding embodiments, wherein the task component is configured to provide a plurality of tasks wherein each of the tasks of the plurality of tasks has a predetermined reinforcer value and wherein the tasks are provided based on a predetermined stimulus type profile.
S88. System according to any of the preceding embodiments, wherein the task component is configured to select stimulus type profile from a plurality of stimulus type profiles.
S89. System according to any of the preceding embodiments, wherein the task component is configured to generate a task based on a reward task template, preferably wherein the task template comprises a predetermined sequence of task conditions.
S90. System according to any of the preceding embodiments, wherein the task component is configured to select a reward task template from a plurality of task templates.
S91. System according to the preceding embodiment, wherein the task component is configured to select a task template and/or a task based on biomarker data and/or a biomarker response profile captured in response to a previously performed task.
S92. System according to the preceding embodiment, wherein the task component is configured to select a task template and/or a task based on a predetermined expected quantitative difference in reinforcer value of the respective stimulus type of the respective task template and/or task, in particular in reference to a previously and/or subsequently provided task.
S93. System according to the preceding embodiment, wherein the task component is configured to provide a fixation task, in particular as part of the plurality of tasks.
S94. System according to any of the preceding embodiments, wherein the biomarker corresponds to a pupil size of the user, and wherein the capture component comprises a camera module (104) configured to capture an image of the eye of the user, preferably the iris of the user, and to determine the pupil size based on the captured image.
S95. System according to any of the preceding embodiments, wherein the capture component comprises an illuminator configured to illuminate part of the user, preferably configured to illuminate the face and/or the eye of the user, more preferably configured to illuminate the iris and/or pupil of the user.
S96. System according to the preceding embodiment, wherein the illuminator is configured to illuminate both eyes, irises and/or pupils of the user.
S97. System according to any of the preceding embodiments comprising features of S95, wherein the illuminator is configured to emit light in the visible spectrum and/or the infrared spectrum, preferably within the range of 700 nm to 1000 nm.
S98. System according to any of the preceding embodiments, wherein the capture component is configured to continuously determine the biomarker with a sampling rate greater or equal to a predetermined threshold rate of 1 Hz, 10 Hz, 25 Hz, 50 Hz or 100 Hz.
S99. System according to any of the preceding embodiments, wherein the capture component is configured to determine a relative value change of the biomarker.
S100. System according to any of the preceding embodiments, wherein the capture component comprises a sensor configured to capture the biomarker data, in particular wherein the processing component is configured to determine the biomarker with a relative tolerance of up to 5%, preferably up to 1%, more preferably only up to 0.1% based on the biomarker data.
S101. System according to any of the preceding embodiments, wherein the capture component is configured to capture the biomarker during a time interval associated with the reward task.
S102. System according to any of the preceding embodiments, wherein the capture module is configured to determine the biomarker for a first time interval prior to the task being provided to the user, for a second time interval during which the task is provided to the user and/or for a third time interval after the reward task is no longer provided to the user.
S103. System according to any of the preceding embodiments comprising features of S100, wherein the sensor is configured to track a pupil size of the user.
S104. System according to any of the preceding embodiments comprising features of S100, wherein the sensor is configured to track an eye movement of the user.
S105. System according to any of the preceding embodiments, wherein the processing component is configured to determine a focus area based on the eye movement, preferably wherein the processing component is configured to determine if the focus area coincides with a display area providing the task or at least partially coincides with the display area.
S106. System according to any of the preceding embodiments, comprising a display component (107) configured to display the task to the user.
S107. System according to the preceding embodiment, comprising a headset component (108) configured to mount onto the head of a user and to dispose the display component at least partially in the field of vision of the user, preferably to dispose a primary area defined by the task within the field of vision of the user.
S108. System according to the preceding embodiment, wherein the headset component is configured to at least partially block ambient light from entering the field of vision of the user.
S109. System according to any of the preceding embodiments, wherein the display component comprises two display units configured to display the task in conjunction with one another, where each of the display units is associated with one eye of the user.
S110. System according to any of the preceding embodiments, comprising a calibration component (109) configured to determine an anhedonia measure and/or anticipatory arousal level of a provided reward task.
Sill. System according to the preceding embodiment, wherein the calibration component is configured to determine a baseline level of the biomarker associated with a baseline level.
S112. System according to any of the preceding embodiments comprising features of S110, wherein the calibration component is configured to determine a maximum level of the biomarker associated with a maximum response.
S113. System according to any of the preceding embodiments comprising features of S110, wherein the calibration component is configured to determine at least one intermediate level of the biomarker associated with a corresponding intermediate response.
S114. System according to any of the preceding embodiments comprising features of S110, wherein the calibration component is configured to generate, in particular to interpolate and/or to extrapolate, a variety of reinforcer values, in particular wherein the stimulus type predictive of a certain reinforcer value corresponds a representative biomarker value, and/or profile parameter.
S115. System according to the preceding embodiment, wherein the task component is configured to provide a set of tasks, wherein the reinforcer value of subsequent tasks is predetermined to increase or to decrease, preferably by a predetermined value, based on the scale and/or category of reinforcer value levels.
S116. System according to the preceding embodiment comprising features of S71, wherein the processing component is configured to determine the profile parameter based on the difference in stimulus type in subsequent tasks.

Below, method embodiments will be discussed. These embodiments are abbreviated by the letter "M" followed by a number. Whenever reference is herein made to "method embodiments", these embodiments are meant.
M1. Method for assessing anhedonia, comprising
   providing a task, in particular a reward task to a user;
   capturing biomarker data of a biomarker of the user in response to the task; and
   generating a biomarker response profile based on the biomarker data.
M2. Method according to the preceding embodiment, comprising the step of determining a profile parameter based on the biomarker response profile and assessing anhedonia, preferably the presence or absence, the severity of anhedonia, basic arousal and/or anticipatory arousal, of the user based on the profile parameter.
M3. Method according to any of the preceding embodiments, comprising the step of providing a stimulus perceivable by the user.
M4. Method according to any of the preceding embodiments with features of M3, wherein the stimulus comprises at least one of a geometric pattern, an image, preferably an image of an object, a symbol, and/or an auditory stimulus.
M5. Method according to any of the preceding embodiments with features of M3, comprising the step of providing the stimulus for a predetermined time interval, in particular wherein the stimulus has a duration of at least 1 second, preferably a duration of 4 to 8 seconds.
M6. Method according to any of the preceding embodiments with features of M3, comprising the step of providing a plurality of stimuli, preferably providing a set of stimuli comprising up to 15 stimuli.
M7. Method according to any of the preceding embodiments with features of M6, wherein each stimulus of the plurality of stimuli corresponds to a predetermined reinforcer value, preferably wherein the reinforcer values of the stimuli differ substantially.
M8. Method according to any of the preceding embodiments with features of M6, wherein the reinforcer value and/or the expectancy value of the predictive stimulus varies according to stimulus category and the eventual biomarker response of the user.
M9. Method according to any of the preceding embodiments, comprising the step of providing a trigger signal, in particular wherein the trigger signal differs substantially in perception by the user with respect to the stimulus.
M10. Method according to any of the preceding embodiments with features of M9, comprising the step of providing the trigger signal after the stimulus, preferably directly after the stimulus or after a pause.
M11. Method according to any of the preceding embodiments with features of M9, wherein the trigger signal or the end of the trigger signal defines the start of a response interval in which a stimulus response by the user is captured.
M12. Method according to any of the preceding embodiments with features of M9, wherein the trigger signal comprises a visual and/or auditory signal, in particular a light flash.
M13. Method according to any of the preceding embodiments with features of M3, comprising the step of capturing the stimulus response and/or initiating the stimulus response by the trigger signal being provided.
M14. Method according to any of the preceding embodiments with features of M13, wherein the stimulus response comprises at least one of
   a motor response;
   an auditory, respectively verbal response;
   an expression response; and/or
   a neural response.
M15. Method according to any of the preceding embodiments with features of M6, comprising the step of capturing a stimulus response for each provided stimulus.
M16. Method according to any of the preceding embodiments with features of M15, comprising the step of assessing the stimulus response based on the provided stimulus according to a set of task rules.
M17. Method according to any of the preceding embodiments with features of M16, wherein assessing the stimulus response comprises matching the stimulus response to the stimulus.
M18. Method according to any of the preceding embodiments with features of M16, comprising the step of determining a response time, preferably a time interval between the stimulus and the stimulus response by the user.
M19. Method according to any of the preceding embodiments with features of M18, comprising the step of matching the stimulus response to the stimulus based on the time interval.
M20. Method according to any of the preceding embodiments, comprising the step of providing a reward to the user based on a reward condition being met.
M21. Method according to any of the preceding embodiments with features of M20, comprising the step of providing a reward associated with a primary reinforcer.
M22. Method according to any of the preceding embodiments with features of M20, comprising the step of providing a reward associated with a secondary reinforcer.
M23. Method according to any of the preceding embodiments with features of M20, wherein the reward condition comprises at least one of
   the time interval not exceeding a maximum threshold value;
   the stimulus response corresponding to an expected stimulus response type
   a stimulus response pattern corresponding to an expected stimulus pattern (e.g., a similarity to norm response);
   the stimulus response adhering to the task rules.
M24. Method according to any of the preceding embodiments with features of M20, comprising the step of providing at least one of the following rewards:
   - a primary reward;
   - a secondary reward, such as a symbol or image, a monetary reward, preferably an auditory and/or visual confirmation of the acquisition of the monetary reward by the user, and a predetermined number of points.
M25. Method according to any of the preceding embodiments with features of M20, comprising the step of providing the reward condition as part of the task to the user, preferably prior to the task being provided.
M26. Method according to any of the preceding embodiments, wherein the task comprises a reward sequence for capturing biomarker data in relation to an anticipated reward and/or a control sequence for capturing biomarker data in relation to anticipating a different reward of different value and/or a passive sequence for capturing biomarker data in relation to the lack of a trigger and/or reward.
M27. Method according to any of the preceding embodiments, wherein the reward sequence comprises a reward stimulus, wherein the reward stimulus indicates the potential for receiving a reward to the user.
M28. Method according to any of the preceding embodiments with features of M27, wherein the reward sequence comprises a reward trigger at a predetermined time interval after the reward stimulus.
M29. Method according to any of the preceding embodiments with features of M27, wherein the task component is configured to provide the reward trigger within a predetermined time interval after the reward stimulus.
M30. Method according to any of the preceding embodiments with features of M27, comprising the step of applying a predetermined control reward condition when processing the captured stimulus response, preferably wherein the predetermined control reward condition comprises a response time captured below a maximum response time threshold value.
M31. Method according to any of the preceding embodiments, and wherein the control sequence comprises a control stimulus, wherein the control stimulus indicates that there is the potential for the user to receive a reward of lesser value, e.g. positive verbal feedback, a checkbox or other image.
M32. Method according to any of the preceding embodiments with features of M29 and M31, wherein the control sequence comprises a control trigger at a predetermined time after the control stimulus.
M33. Method according to any of the preceding embodiments with features of M31, comprising the step of providing the control trigger within a predetermined time interval after the control stimulus.
M34. Method according to any of the preceding embodiments with features of M31, wherein the passive sequence comprises a passive stimulus, wherein the passive stimulus indicates the absence of a trigger signal and that there is no potential for the user to receive a reward.
M35. Method according to any of the preceding embodiments with features of M31, wherein the passive sequence comprises providing a trigger signal and wherein the passive stimulus indicates to the user that the trigger signal will follow, in particular wherein the trigger signal is provided after the passive stimulus, and wherein the passive stimulus further indicates that there is no potential for the user to receive a reward.
M36. Method according to any of the preceding embodiments with features of M27 and M31, comprising the step of providing an identical signal as the response trigger and the control trigger.
M37. Method according to any of the preceding embodiments with features of M27 and M31, comprising the step of providing a first stimulus as the reward stimulus and a further stimulus as the control stimulus, and wherein the first stimulus and the further stimulus are distinguishable with respect to one another by the user.
M38. Method according to any of the preceding embodiments, wherein the reward stimulus, the control stimulus and/or the passive stimulus are impact neutral with respect to the biomarker data, biomarker response profile and/or profile parameter when being perceived by the user without reward context.
M39. Method according to any of the preceding embodiments, wherein the reward stimulus, the control stimulus and/or the passive stimulus can be geometric transformations with respect to one another, in particular when being perceived by the user without reward context.
M40. Method according to any of the preceding embodiments, wherein the reward stimulus, the control stimulus and/or the passive stimulus are defined within a predetermined biomarker response value interval.
M41. Method according to any of the preceding embodiments, comprising the step of spatially filtering the reward stimulus, the control stimulus and/or the passive stimulus.
M42. Method according to any of the preceding embodiments, comprising the step of determining and/or removing effects of the reward stimulus and/or the control stimulus from the biomarker data, preferably removing signal cross-contamination from the stimulus into the biomarker data.
M43. Method according to any of the preceding embodiments, comprising the step of providing a segmented task comprising at least one of:
   an anticipation phase, which preferably comprises the reward stimulus, the control stimulus and/or the passive stimulus;
   a response trigger phase;
   a response capture phase;
   a feedback phase;
   a reward consumption phase; and/or
   a fixation phase.
M44. Method according to any of the preceding embodiments with features of M43, comprising the step of capturing biomarker data during at least one phase of the segmented task, preferably during each phase of the segmented task.
M45. Method according to any of the preceding embodiments, comprising the step of synchronizing capturing the biomarker data with the reward stimulus, preferably such that a time interval before, during and/or after the reward stimulus is captured.
M46. Method according to any of the preceding embodiments, comprising the step of synchronizing capturing the biomarker data with the control stimulus, preferably such that a time interval before, during the secondary control stimulus is captured.
M47. Method according to any of the preceding embodiments, wherein the biomarker data comprises a biomarker vector, preferably a pupil size vector.
M48. Method according to any of the preceding embodiments with features of M47, comprising the step of determining a derivative of the biomarker vector, preferably a first derivative and/or a second derivative.
M49. Method according to any of the preceding embodiments comprising the step of applying a fit to at least part of the biomarker vector, in particular a linear or non-linear fit.
M50. Method according to any of the preceding embodiments with features of M49, comprising the step of segmenting the biomarker vector and applying a fit to at least one segment of the biomarker vector, preferably applying differing fits to different segments of the biomarker vector.
M51. Method according to any of the preceding embodiments with features of M49, comprising the step of determining a profile parameter based on the fit, preferably determining a fit characteristic, e.g., an intercept value and/or an acceleration value, of the biomarker vector.
M52. Method according to any of the preceding embodiments with features of M49, comprising the step of applying the fit time-bin-wise to the biomarker vector, preferably wherein each bin represents a predetermined time interval, in particular a time-interval between 0.5-2.0 s.
M53. Method according to any of the preceding embodiments with features of M52, wherein the segment corresponds to at least one of the following:
   a first time interval starting with the reward stimulus or the control stimulus, preferably wherein the first time interval is binned, in particular binned in 1 s i nterva ls;
   an onset time interval starting at a predetermined time, preferably between 100 ms and 2000 ms, more preferably 500 ms or 1000 ms before the reward trigger, respectively the control trigger;
   a finalizing time interval ending with the subsequent stimulus, preferably starting 500 ms and 5000 ms, more preferably 2000 ms before the subsequent stimulus;
   a predetermined segment between the stimulus and the subsequent stimulus; the full stimulus duration.
M54. Method according to any of the preceding embodiments comprising the step of repeating a task for a plurality of instances.
M55. Method according to any of the preceding embodiments with features of M54, comprising the step of comparing stimuli responses based on the plurality of captured biomarker responses corresponding to the respective task instances.
M56. Method according to any of the preceding embodiments with features of M54, comprising the step of wherein the processing component is configured to determine the profile parameter for each task instance and to compare the profile parameter across the task instances.
M57. Method according to any of the preceding embodiments with features of M56, comprising the step of determining an anhedonia measure and/or anticipatory arousal level, in particular by matching the biomarker profile parameter to the stimulus types, in particular with a quantitative or qualitative scale.
M58. Method according to any of the preceding embodiments, comprising the step of repeating a task or a task component for a predetermined number of times.
M59. Method according to any of the preceding embodiments with features of M58, comprising the step of providing the repeated tasks or task components in a predetermined sequence, in particular in a predetermined sequence of stimuli, preferably repeating stimuli.
M60. Method according to any of the preceding embodiments, comprising the step of training a machine learning model based on the biomarker training data, wherein the machine learning model is adapted to identify signal features that characterize anhedonia in the user.
M61. Method according to any of the preceding embodiments with features of M60, comprising the step of applying the machine learning model to biomarker data and to output of the presence or absence and/or severity of anhedonia of the user based on the signal features.
M62. Method according to any of the preceding embodiments with features of M60, wherein the machine learning model is a neural network that comprises one or more hidden layers and an output layer, wherein the output layer provides a probability distribution over different levels of anhedonia.
M63. Method according to any of the preceding embodiments comprising the step of providing task data to the task component, modifying the current reward task based on the task data and performing a cross-validation on the trained machine learning model using a validation set of biomarker data, wherein the cross-validation comprises evaluating the performance of the machine learning model on the validation set and adjusting one or more parameters of the machine learning model based on the evaluation.
M64. Method according to any of the preceding embodiments with features of M60, comprising the step of performing a feature extraction on the biomarker data, wherein the feature extraction comprises applying one or more filters and/or transformations to the biomarker data to obtain a reduced set of features that are relevant for anhedonia identification.
M65. Method according to any of the preceding embodiments with features of M61, comprising the step of applying the machine learning model to the preprocessed biomarker data and to output a level of anhedonia in the subject based on the signal features.
M66. Method according to any of the preceding embodiments, wherein the profile parameter corresponds to a statistical measure and/or a parameter of a mathematical function, in particular a function fitted to the biomarker response profile.
M67. Method according to any of the preceding embodiments with features of M2, comprising the step of determining a difference measure and to assess the presence or absence and/or severity of anhedonia of the user based on the difference measure.
M68. Method according to any of the preceding embodiments, wherein the difference measure corresponds to at least one of the following:
   difference of the profile parameter to a predetermined baseline value;
   difference of a fitted curve parameter to a predetermined fitted curve parameter;
   similarity of the biomarker response profile to a reference profile;
   scaling of the biomarker response profile in comparison to a reference profile;
   scaling of a parameter fitted to the biomarker response profile in comparison to a reference parameter.
M69. Method according to any of the preceding embodiments with features of M66, comprising the step of determining an anhedonia measure based on the biomarker response profile and/or the difference measure.
M70. Method according to any of the preceding embodiments, comprising the step of comparing the difference measure and/or the biomarker response profile against a set of predetermined values and associating the biomarker response profile with an anhedonia class, which in particular corresponds to a pathological anhedonia of the user.
M71. Method according to any of the preceding embodiments, comprising the step of mapping the profile parameter to a value on an anhedonia scale to provide an anhedonia measure.
M72. Method according to any of the preceding embodiments, comprising the step of updating the anhedonia measure while the task is provided, preferably while the reward task is performed by the user.
M73. Method according to any of the preceding embodiments, comprising the step of updating the biomarker response profile periodically or continuously.
M74. Method according to any of the preceding embodiments, comprising the step of updating the biomarker response profile while the reward task is provided, preferably while the reward task is performed by the user.
M75. Method according to any of the preceding embodiments with the features of M69, comprising the step of updating the anhedonia measure periodically or continuously, in particular when a task is completed, a sub-part of the task is completed or at least part of the task is completed.
M76. Method according to any of the preceding embodiments, comprising the step of providing the anhedonia measure, in particular to a review component.
M77. Method according to any of the preceding embodiments, comprising the step of providing a confidence measure corresponding to a variability of the anhedonia measure, in particular a variance and/or error measure.
M78. Method according to any of the preceding embodiments, comprising the step of providing a representation of the biomarker response profile, in particular a numerical and/or graphical representation.
M79. Method according to any of the preceding embodiments, comprising the step of providing a representation of the anhedonia measure and/or the confidence measure.
M80. Method according to any of the preceding embodiments, comprising the step of providing the representation of the anhedonia measure periodically or continuously, in particular parallel to the task component providing the reward task.
M81. Method according to any of the preceding embodiments with features of M78, comprising the step of providing task data to the task component and wherein the task component is configured to modify the current reward task based on the task data.
M82. Method according to any of the preceding embodiments with features of M78, comprising the steps of adjusting a reinforcer value of a given stimulus type of the reward task and/or providing a subsequent task with a reinforcer value different to the reinforcer value of the previous task.
M83. Method according to any of the preceding embodiments, comprising the step of storing a plurality of biomarker response profiles and/or anhedonia measures.
M84. Method according to any of the preceding embodiments, comprising the steps of comparing a present biomarker response profile to the plurality of response profiles and generating a relative measure of the biomarker response profile indicating a relation of the present biomarker response profile to the plurality of response profiles.
M85. Method according to any of the preceding embodiments with features of M83, comprising the steps of comparing a present anhedonia measure to the plurality of anhedonia measures and generating a measure, in particular a relative measure, of the present anhedonia measure indicating a relation of the present anhedonia measure to the plurality of anhedonia severity measures
M86. Method according to any of the preceding embodiments, comprising the steps of receiving medical data relating to the user to which the task is provided and providing a relevance indicator corresponding to the anhedonia measure in relation to the medical data.
M87. Method according to any of the preceding embodiments, comprising the step of providing a plurality of tasks wherein each of the tasks of the plurality of tasks has a predetermined reinforcer value and wherein the tasks are provided based on a predetermined stimulus type profile.
M88. Method according to any of the preceding embodiments, comprising the step of selecting a stimulus type profile from a plurality of stimulus type profiles.
M89. Method according to any of the preceding embodiments, comprising the step of generating a task based on a reward task template.
M90. Method according to any of the preceding embodiments, comprising the step of selecting a reward task template from a plurality of task templates.
M91. Method according to any of the preceding embodiments, comprising the step of selecting a task template and/or a task based on biomarker data and/or a biomarker response profile captured in response to a previously performed task.
M92. Method according to any of the preceding embodiments, comprising the step of selecting a task template and/or a task based on a predetermined expected quantitative difference in reinforcer value of the respective stimulus type of the respective task template and/or task, in particular in reference to a previously and/or subsequently provided task.
M93. Method according to any of the preceding embodiments, comprising the step of providing a fixation task, in particular as part of the plurality of tasks.
M94. Method according to any of the preceding embodiments, wherein the biomarker corresponds to a pupil size of the user, and comprising the steps of capturing an image of the eye of the user, preferably the iris of the user, and determining the pupil size based on the captured image.
M95. Method according to any of the preceding embodiments, comprising the step of illuminating part of the user, preferably illuminating the face and/or the eye of the user, more preferably illuminating the iris and/or pupil of the user.
M96. Method according to any of the preceding embodiments, comprising the step of illuminate both eyes, irises and/or pupils of the user.
M97. Method according to any of the preceding embodiments, comprising the step of emitting light in the visible spectrum and/or the infrared spectrum, preferably within the range of 700 nm to 1000 nm.
M98. Method according to any of the preceding embodiments, comprising the step of continuously determining the biomarker with a sampling rate greater or equal to a predetermined threshold rate of 1 Hz, 10 Hz, 25 Hz, 50 Hz or 100 Hz.
M99. Method according to any of the preceding embodiments, comprising the step of determining a relative value change of the biomarker.
M 100. Method according to any of the preceding embodiments, comprising the step of capturing the biomarker data, in particular determining the biomarker with a relative tolerance of up to 5%, preferably up to 1%, more preferably only up to 0.1% based on the biomarker data.
M101. Method according to any of the preceding embodiments, comprising the step of capturing the biomarker during a time interval associated with the reward task.
M 102. Method according to any of the preceding embodiments, comprising the step of determining the biomarker for a first time interval prior to the task being provided to the user, for a second time interval during which the task is provided to the user and/or for a third time interval after the reward task is no longer provided to the user.
M103. Method according to any of the preceding embodiments with features of M100, comprising the step of tracking a pupil size of the user.
M104. Method according to any of the preceding embodiments with features of M100, comprising the step of tracking an eye movement of the user.
M105. Method according to any of the preceding embodiments, comprising the step of determining a focus area based on the eye movement, preferably determining if the focus area coincides with a display area providing the task or at least partially coincides with the display area.
M106. Method according to any of the preceding embodiments, comprising the step of displaying the task to the user.
M107. Method according to any of the preceding embodiments, comprising the step of mounting a headset component onto the head of a user and disposing the display component at least partially in the field of vision of the user, preferably disposing a primary area defined by the task within the field of vision of the user.
M108. Method according to any of the preceding embodiments, comprising the step of at least partially blocking ambient light from entering the field of vision of the user.
M109. Method according to any of the preceding embodiments, comprising the step of displaying the task by means of two display units in conjunction with one another, wherein each of the display units is associated with one eye of the user.
M110. Method according to any of the preceding embodiments, comprising the step of determining an anhedonia measure and/or anticipatory arousal level of a provided reward task.
Mill. Method according to any of the preceding embodiments, comprising the step of determining a baseline level of the biomarker associated with a baseline level.
M112. Method according to any of the preceding embodiments with features of M110, comprising the step of determine a maximum level of the biomarker associated with a maximum response.
M113. Method according to any of the preceding embodiments with features of M110, comprising the step of determining at least one intermediate level of the biomarker associated with a corresponding intermediate response.
M114. Method according to any of the preceding embodiments with features of M110, comprising the step of generating, in particular interpolating and/or extrapolating, a variety of reinforcer values, in particular wherein the stimulus type is predictive of a certain reinforcer value corresponding to a representative biomarker value, and/or profile parameter.
M115. Method according to the preceding embodiment, comprising the step of providing a set of tasks, wherein the reinforcer value of subsequent tasks is predetermined to increase or to decrease, preferably by a predetermined value, based on the scale and/or category of reinforcer value levels.
M116. Method according to the preceding embodiment with features of M71, comprising the step of determining the profile parameter based on the difference in stimulus type in subsequent tasks.

Below, embodiments of a computer program product will be discussed. These embodiments are abbreviated by the letter "C" followed by a number. Whenever reference is herein made to the "computer program product embodiments", these embodiments are meant.
C1. A computer program product comprising instructions, which, when executed by a system and any of its components according to any of the system embodiments, cause the system and its components to perform the steps, for which the system and/or any of its components are configured.
C2. A computer program product comprising instructions, which, when executed by the system and any of its components according to any of the system embodiments, cause the system and its respective components to perform the steps of the method according to any of the method embodiments, which method steps are performed by the system and/or the respective components according to the method.

Below, use embodiments will be discussed. These embodiments are abbreviated by the letter "U" followed by a number. Whenever reference is herein made to "use embodiments", these embodiments are meant.
U1. Use of the system according to any of the preceding system embodiments, for carrying out the method according to any of the preceding method embodiments.
U2. Use of the method according to any of the preceding method embodiments and the system according to any of the preceding embodiments, for detection of anhedonia, in particular in users with hypo-arousal, specifically impairments due to psychopathological syndromes.
U3. Use of the system or method according to any of the preceding system or method embodiments for carrying out a diagnosis and/or therapy of a patient.
U4. Use of the system or method according to any of the preceding system or method embodiments for carrying out a diagnosis and/or therapy of a patient in connection with anhedonia, in particular in patients with hypo-arousal, specifically impairments due to psychopathological syndromes.

### Brief description of figures

The present invention will now be described with reference to the accompanying drawings, which illustrate embodiments of the invention. These embodiments should only exemplify, but not limit, the present invention.
- Fig. 1: schematically depicts a drawing of an embodiment of a system according to the invention;
- Fig. 2: schematically depicts a task sequence according to the invention;
- Fig. 3A: schematically depicts representations of linear fitting functions of the biomarker data according to the invention; and
- Fig. 3B: schematically depicts representations of non-linear fitting functions of the biomarker data according to the invention.

### Preferred embodiments

It is noted that not all the drawings carry all the reference signs. Instead, in some of the drawings, some of the reference signs have been omitted for sake of brevity and simplicity of illustration. Embodiments of the present invention will now be described with reference to the accompanying drawings.

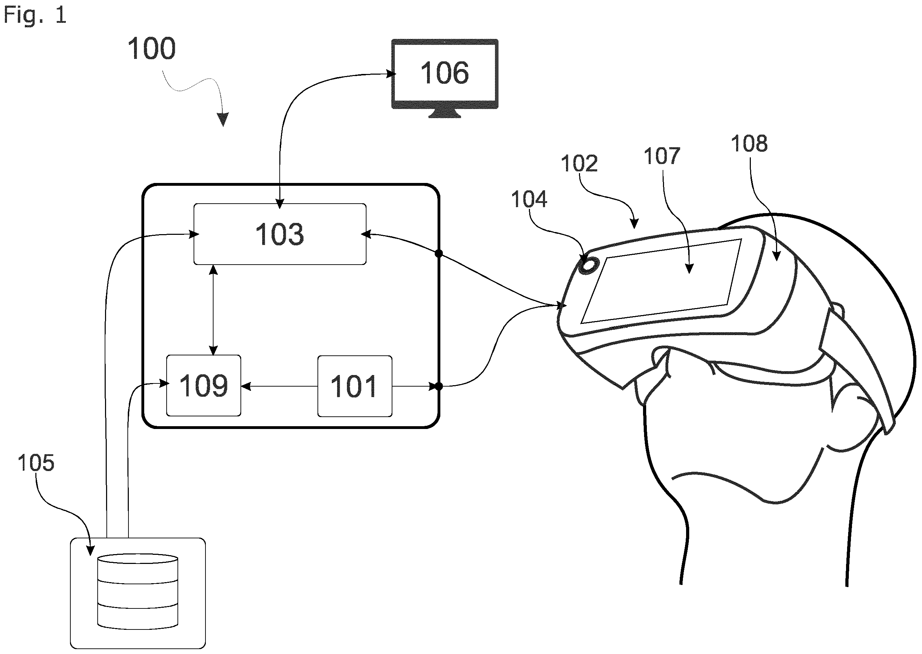

**Figure 1** shows a schematic representation of the system 100 for assessing anhedonia in depression according to the invention. The physiological process of interest can be described as reward anticipatory hypo-arousal. The system 100 can be configured to present any perceivable stimulus that requires a motor response at its offset, preferably with a light flash at offset, but other forms of stimuli (images of objects, symbols and auditory) can be used. The stimulus can be of any length. Preferably, the stimulus is shown for a duration of at least of 1 s to 2 s, preferably 4 s to 8 s. Longer durations are also possible.

The stimulus can be presented multiple times in a row, preferably 3 to 5 times, or up to 10 times. Two control stimuli follow similar parameters: one control stimulus is also followed by a light flash and/or another visual/auditory stimulus at offset and can require a motor response within a predetermined reaction interval; another may or may not be followed by any stimulus and may or may not require a motor response, but there is no potential to win a reward. The goal of the task for users can be defined as tapping a screen, e.g. a smartphone screen, sufficiently fast to get the associated reward, e.g. a checkmark symbol, a monetary incentive (+1 euro) or any other bonus points. Preferably monetary or bonus points incentives can be restricted to the first stimulus, the reward stimulus, and the checkmark stimulus can follow the control stimulus requiring a motor response. Stimuli can be spatially filtered to prevent a pupillary light reflex just after stimulus offset.

The capture component can be a front facing camera of a smart device, e.g., smartphone. Preferably, the front facing camera can be combined with a (near) infrared illuminator to achieve a high-quality image of the pupil, after which the pupil diameter can be obtained through ellipse-fitting, a process which can be supported by (non-) linear models such as a neural network. Preferably, a head fixation using a VR/AR system can be used. Alternatively, a full VR/AR headset can be used.

The time-window of interest can be the duration of the reward and control stimuli, i.e. the anticipatory window before the subsequent light flash or other stimulus requiring the motor response. Other aspects of the task can be captured and analyzed as well, with a particular interest in the light response, the reward consumption phase (after winning a small monetary sum) and/or the fixation phase.

The system can comprise a task component 101, a capture component 102, and a processing component 103. The task component can be configured to provide a task to the user. The capture component can be configured to capture biomarker data of a biomarker of the user, i.e., a pupil dilation. The biomarker can also correspond to brain activity, eye movement, heart rate, actigraphy, and/or skin conductance. Actigraphy can be performed by the VR headset component. The processing component can be configured to generate a biomarker response profile based on the biomarker data. The biomarker response profile reflects how the user's biomarker changes in response to the cognitive task.

The processing component is further configured to assess a level of anhedonia, respectively the presence or absence of anhedonia based on the biomarker response profile. To do so, the processing component can be configured to perform one or more of the following functions:
- Determining a profile parameter based on the biomarker response profile and compare it to a predetermined baseline value. The profile parameter can be a statistical measure (such as mean, standard deviation, or correlation) or a parameter of a mathematical function (such as slope, intercept, or curvature) fitted to the biomarker response profile. The difference between the profile parameter and the baseline value can indicate the level of anhedonia of the user. Preferably, the profile parameter can relate to one of the following quantities: variance, standard deviation, first derivative of pupil dilation, first derivative of pupil size. Other measures relevant to the chosen statistical analysis means can be used.
- Determining a difference measure based on the similarity or scaling of the biomarker response profile or a parameter fitted to it in comparison to a reference profile or a reference parameter. The reference profile or parameter can represent a normative or expected biomarker response for a given task. The difference measure can indicate how much the user's biomarker response deviates from the reference.
- Mapping the profile parameter or the difference measure to a value on an anhedonia severity scale to provide an anhedonia measure, respectively a hypo-arousal measure. The hypo-arousal scale can be a numerical or categorical scale that reflects different levels of hypo-arousal, such as normal, mild, moderate, or severe hypo-arousal.
- Comparing the difference measure and/or the biomarker response profile against a set of predetermined values and associate the biomarker response profile with a hypo-arousal or anhedonia class. The anhedonia class can correspond to a pathological impairment of the user.

The processing component can update the biomarker response profile, the difference measure, and/or the anhedonia measure periodically or continuously while the task is provided or performed by the user. This can allow for real-time monitoring and assessment.

The system can also comprise a review component 106 configured to provide a representation of the biomarker response profile, the difference measure, and/or the anhedonia measure. The representation can be numerical and/or graphical and can be provided periodically or continuously, in particular parallel to the task component providing the task. The review component can also provide a confidence measure corresponding to a variability of the anhedonia measure, such as a variance or an error measure. The representation and the confidence measure can help evaluate the reliability and validity of the assessment.

The system can also be configured to provide feedback and adaptation of the task based on the biomarker response profile and/or the anhedonia measure. Thereto, the system can be configured to execute one of the following functions:
- Providing task data to the task component from the review component and modifying the current task based on the task data. The task data can include information about the user's performance, engagement, or preference for the task.
- Adjusting the task, i.e. a response window of the current or subsequent task based on the biomarker response profile and/or the motor response timing. The response window can be increased or decreased to match the user's response level and to provide an optimal challenge for the user.

A plurality of biomarker response profiles and/or anhedonia measures can be stored in a database module (105). The presently captured biomarker response profile or anhedonia measure can be compared to stored biomarker response profiles, respectively anhedonia measures. The comparison can generate a relative measure of the present biomarker response profile or anhedonia measure indicating how it relates to the stored ones. The relative measure can help identify trends, patterns, or outliers in the user's anhedonia, respectively hypo-arousal functioning over time or across different tasks.

The system can be configured to receive medical data relating to the user, such as diagnosis, medication, symptoms, and/or other biomarkers, and provide a relevance indicator corresponding to the anhedonia measure in relation to the medical data. The relevance indicator can help evaluate how the user's anhedonia is affected by or affects their medical condition.

The system can also be configured to provide different types of tasks with different levels of reward anticipation. Reward anticipation can correlate to the level of desirability associated by the user to the task. A first task and a second task can be provided, where the second task is configured to induce a higher reward anticipation than the first task.

Tasks can also be provided according to a predetermined anticipatory reward profile. The anticipatory reward profile can specify how the level of an expected anticipation arousal changes over time or across tasks. For example, the anticipatory reward profile can be increasing, decreasing, constant, variable, pseudo-randomly or randomly interspersed. The system can select an anticipatory reward profile from a plurality of predefined profiles or generate a custom profile based on user input or feedback.

Tasks can be generated based on a task template that can define the structure, content, and parameters of a task. For example, the task template can specify what type of stimuli (e.g., words, numbers, images, sounds) are used, how they are presented (e.g., duration, frequency, order), and what type of response (e.g., verbal, manual, eye movement) is required from the user. The system can select a task template from a plurality of predefined templates or generate a custom template based on user input or feedback.

The system can be configured to tailor the task to the user's hypo-arousal state, in particular based on previously captured biomarker data, respectively a previously captured biomarker response profile.

The system can control how much the level of reward anticipation changes between tasks. For example, if the system may be configured to increase or decrease the level of reward anticipation gradually or abruptly.

The system can also be configured to capture different types of biomarkers from different sources. Biomarkers are biological indicators that reflect physiological processes related to neurocognitive functioning.

The system can comprise an illuminator to illuminate part of the user, preferably the face and/or the eye of the user, more preferably the iris and/or pupil of the user. The illuminator can be integrated into the headset component, specifically the illuminator can be part of a VR-headset. The illuminator can help improve the quality and accuracy of the image captured by the camera module. Preferably, the illuminator can be configured to illuminate both eyes, irises, and/or pupils of the user. The illuminator can also be configured to optimize the illumination for one eye, e.g., properties of the illumination such as focal point, brightness, contrast, color and/or brightness gradient can be determined according to the characteristics of a predetermined eye of the user.

The illuminator can be configured to emit infrared light that can help reduce the interference from ambient light and increase the contrast between the iris and the pupil.

The processing component can be configured to normalize biomarker data across different users or sessions and account for individual differences or variations in baseline levels or measurement conditions.

The system can also be configured to provide an immersive and interactive experience for the user by using a virtual reality (VR) headset. The VR headset can create a simulated environment that surrounds and responds to the user's actions. The system can comprise a display component (107) to display the cognitive task to the user. The display component can present visual stimuli that are relevant to the task, such as words, numbers, images, or shapes. The display component can also provide feedback or instructions to guide or motivate the user during or after the task.

A headset component (108) can mount onto the head of the user and dispose the display component at least partially in the field of vision of the user, preferably to dispose a primary area defined by the task within the field of vision of the user. The headset component can help position and stabilize the display component in relation to the user's eyes and head movements. The headset component can also help isolate or block ambient light from entering the field of vision of the user and reduce distractions or interference from the external environment.

The display component can be comprised of two display units that can create a stereoscopic effect that enhances the depth perception and/or increases realism of the visual stimuli. For example, the displayed task may be perceived as a three-dimensional representation. The VR headset can immerse the user in a realistic and interactive environment. The system can also use the feedback component to provide the patient with adaptive and personalized training tasks that target their specific cognitive impairments and improve their functional outcomes.

The system may also comprise a heart rate monitor, a skin conductance sensor and/or an actigraphy, i.e., an actimetry sensor, to capture the user's heart rate, skin conductance and/or motor activity as biomarkers of emotional arousal and anxiety. The system can apply a correction and/or weight to the biomarker response profile according to the additional biomarkers.

The system can comprise a smart device which is used as an output interface for the task component, respectively the processing component. The smart device may comprise sensors, in particular imaging devices, configured to capture images of the pupil. A local processor of the smart device can be configured to preprocess the captured data. The preprocessed data may be provided to the processing component for analysis and generating the biomarker response profile. Herein, preprocessing may increase the data quality, i.e. an SNR increase, image processing and/or may increase data transfer efficiency in compressing the captured data prior to transfer of the data to the processing component. Alternatively, the local processor of the smart device can be the processing component such that evaluating the biomarker data can be achieved locally. The preprocessing and/or processing of the data may comprise interpolation to account for missing or low-quality data, e.g., caused by blinks, and/or further comprise smoothing, filtering and/or interpolation of the data and/or standardization, e.g., z-scoring.

The system may comprise a decentralized communication infrastructure. Specifically, the processing component, task component and/or database module can be cloud based or remotely located components.

The processing component can be configured to determine an inconsistency in the biomarker data, i.e., blinking when capturing pupil dilation. The processing component can be configured to adjust the task based on detecting an inconsistency. For example, the processing component can provide task data to the task component, wherein the task data indicates a repeat section of the corresponding task section that was affected by the inconsistency.

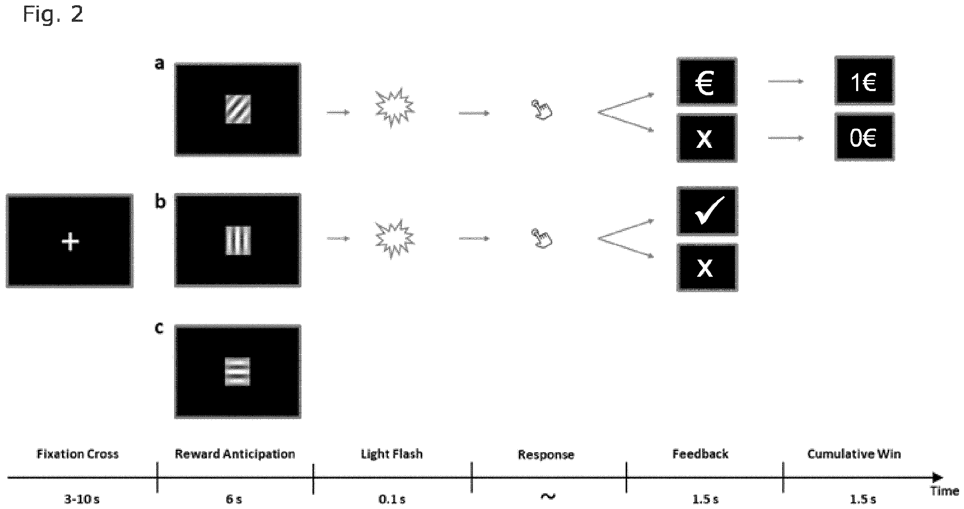

**Figure 2** shows a schematic representation of a task sequence according to the invention. A task can start with a fixation cross, which can be followed by a Gabor patch, in particular one of three Gabor patches corresponding each to a specific condition one of three. Both (a) reward condition and (b) neutral condition can be followed by a light flash to which a user has to respond as fast as possible. Based on the speed of the response specific feedback is provided indicating if the user was fast enough or too slow. Given the chance to win 1 euro per trial in the reward condition, the cumulative total win can be presented only in this condition. (c) can be the control condition in which only the Gabor patch is presented and no response is required.

Depending on individual reaction times together with an adaptive algorithm allowing for approximately 50% success rate per user across all trials, either positive or negative feedback can be displayed for 1.5 s. In the neutral condition, this feedback can be either a green checkmark (i.e., fast response) or a red cross (i.e., too slow response). In the reward condition the feedback can consist of a green euro sign if responses were fast enough and a red cross if they were not. In the reward condition, another feedback can be shown for 1.5 s that provided the current cumulative total win at that time point in the task. Each user can complete 30 trials in total (i.e., 10 trials per condition) in a pseudo-randomized order.

Pupil diameter can be recorded, in particular a pupil diameter of the right eye with a sampling rate in the range of 30 Hz to 1000 Hz using a camera, in particular a smartphone camera and/or an eye tracker. The eye position can be calibrated by means of a nine-point calibration procedure. Pupil data can be pre-processed and analyzed, including linearly interpolating missing data values due to eye blinks from 100 ms before the start of the blink until 100 ms after the end of the blink, which can be automatically detected. In order to deal with non-biological outliers, the pupil data can be smoothed by using a 200 ms sliding window (100 ms before and after a given data-point), replacing the actual value with the mean value of that window. In a final step, the pupil data can be z-transformed to account for average variability in pupil size across participants.

Pupil data can be discarded based on the following criteria: single trials can be discarded if more than 50% of data values are missing, complete data sets can be removed if missing data values for the entire session exceeded 15% (N = 32). Furthermore, to ensure that pupil data being analyzed mainly originated from users looking at the screen, a rectangular gaze window can be defined. The median for gaze in x and y directions of the entire 6 seconds anticipation window can be computed yielding two center coordinates per user. Those coordinates can be used to determine average standard deviations for the x-y gaze shift coordination pair across all users. Given these parameters, a rectangular gaze window based on 3.3 SD from each participant's center coordinates can be defined. Trials in which user's gaze remains outside this window for more than one second can be removed.

The following analysis steps can be conducted. A first derivative of the pupil time series data of the reward anticipation phase can be computed to obtain pupil dilations for all three conditions (reward, control and passive stimulus). To determine the association between depressive symptom load and reward anticipatory arousal, the correlation between the differential mean score of pupil dilation (reward minus control condition) and the M-CIDI item reflecting the number of depressive symptoms within the last two weeks can be computed. Correlational analysis between differential mean pupil dilation and the number of symptoms can be conducted within a group of depressed users. Mean pupil dilations time courses for the 6s reward anticipation window can be compared between healthy controls and depressed users, as well as between healthy controls and acutely depressed users (defined as having five or more symptoms within the last two weeks). Pupil dilation values for the fixation and reward consumption phases can be extracted. Here, reward consumption can be defined as the feedback phases (with sufficiently quick responses and thus including either a green monetary symbol or green checkmark symbol) in the two reward conditions, that is, (monetary) reward and control (verbal feedback) stimuli.

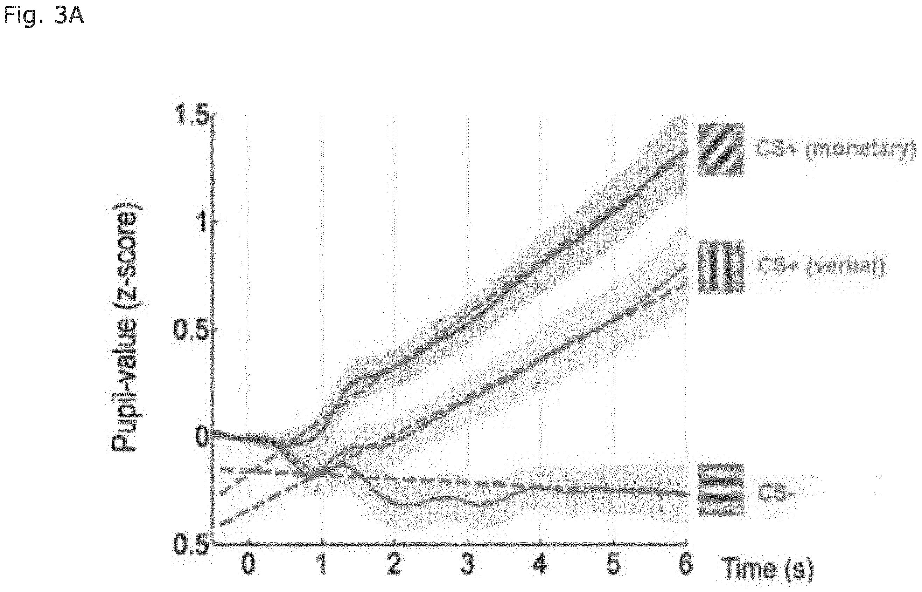

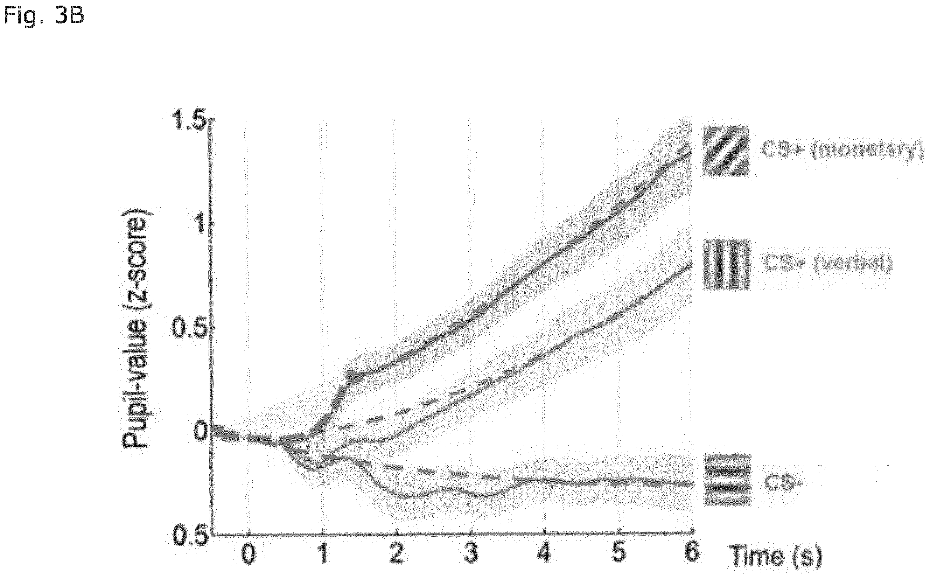

**Figure 3** shows schematic representations of linear **(3A)** and non-linear **(3B)** fitting functions of the biomarker data for full stimulus-windows (dashed lines) or parts of the window (bold dashed line in Fig. 3B). The biomarker data can relate to a monetary reward stimulus "CS+ (monetary)", a neutral stimulus with verbal feedback "CS+ (verbal)" or a control stimulus with no feedback and not requiring a motor response "CS-".

The fit of a linear or non-linear model can be performed on a summarized time-course during the anticipatory time-window, preferably including a brief baseline period that starts 500ms before stimulus onset. This can include the full time-window but also the last part, e.g. the last two seconds, as well as the first part, e.g. the first two seconds, or any partial chopping of the full window of either pupil size and/or its first-order derivative, the pupil dilation, and/or second-order derivative. Preferably, the linear or non-linear function can provide a parameter for the acceleration of the function and an intercept, which can both be relevant depending on the part of time-window being analyzed.

By means of repeating the task, decreasing or increasing pupil values can be tracked over time (repetition) and compared between stimuli. In the example of ten stimulus presentations, also the slope and intercept over the fitted responses per trial (whether mean, median, beta or intercept of pupil size or dilation is used per task) can provide information regarding reward anticipatory hypo-arousal as a marker for anhedonia.

The analysis can comprise within-task fitting and/or across-task fitting. The reward stimulus can be compared to other stimuli and a differential score can be obtained. These differential scores for pupil size and/or dilation, e.g. of reward versus the first or second control stimulus, can represent primary parameters for reward anticipatory hypo-arousal in a given user. These parameters can be compared with a range of values from healthy control groups, depressed groups without reward anticipatory hyperarousal and/or non-depressed groups affected by other relevant physiological processes (e.g. cognitive processes). Reference values can be compared with regards to age, gender and/or education level.

The biomarker score values, i.e. biomarker profile parameters, can have high split-half reliability, i.e. a high Pearson correlation, e.g. of over 0.80, wherein test-retest reliability can alternatively be used as a correlation measure. Thus, these parameters can not only be used to assess anhedonia at baseline for the purposes of treatment allocation, but they can also be applied to monitor disease progression or treatment monitoring, in the early stages and/or for longer duration (e.g. over weeks and months). This can provide a treating clinician with important information about the actual treatment response of psychotherapeutic and pharmacological interventions, but it can also be used to titrate the latter type of treatment with higher frequent tests.

## Claims

1. System (100) for assessing anhedonia, comprising
a task component (101) configured to provide a reward task to a user;
a capture component (102) configured to capture biomarker data of a biomarker of the user in response to the reward task; and
a processing component (103) configured to generate a biomarker response profile based on the biomarker data.

2. System according to claim 1, wherein the task component comprises a presentation module configured to provide a stimulus perceivable by the user, and wherein the task component is configured to provide the stimulus for a predetermined time interval.

3. System according to any of the preceding claims, wherein the task component is configured to provide a trigger signal, and wherein the capture component is configured to capture a stimulus response, and wherein the processing module is configured to determine a time interval between the stimulus and the stimulus response by the user.

4. System according to any of the preceding claims, comprising a reward module configured to provide a reward to the user based on a reward condition being met.

5. System according to any of the preceding claims, wherein the task comprises a reward sequence for capturing biomarker data in relation to an anticipated reward, wherein the reward sequence comprises a reward stimulus and the reward stimulus indicates the potential for receiving a reward to the user, and a control sequence for capturing biomarker data in relation to anticipating a different reward.

6. System according to any of the preceding claims, wherein the biomarker data comprises a biomarker vector, wherein the processing component is configured to segment the biomarker vector and to apply a fit to at least one segment of the biomarker vector, and to determine a profile parameter based on the fit, wherein the processing component is configured to determine the profile parameter for each task instance and to compare the profile parameter across the task instances.

7. System according to claim 6, wherein the profile parameter corresponds to a function fitted to the biomarker response profile.

8. System according to claim 6 or 7 wherein the processing component is configured to determine an anhedonia measure based on the comparison outcome of the profile parameters.

9. System according to any of the preceding claims, wherein the processing component is configured to determine a difference measure and to assess the presence or absence and/or severity of anhedonia of the user based on the difference measure.

10. System according to any of the preceding claims, comprising a machine learning component configured to train a machine learning model based on biomarker training data, wherein the machine learning model is adapted to identify signal features that characterize anhedonia in the user.

11. System according to any of the preceding claims, wherein the task comprises a passive sequence for capturing biomarker data in relation to the lack of a trigger and lack of reward, wherein the passive sequence comprises a passive stimulus, and wherein the passive stimulus indicates the absence of a trigger signal and that there is no potential for the user to receive a reward.

12. System according to any of the preceding claims, wherein the task component is configured to provide a segmented task comprising at least one of:
an anticipation phase, which preferably comprises the reward stimulus, the control stimulus and/or the passive stimulus;
a response trigger phase;
a response capture phase;
a feedback phase;
a reward consumption phase; and/or
a fixation phase.

13. Method for assessing anhedonia, comprising
providing a reward task,
capturing biomarker data of a biomarker of the user in response to the reward task; and
generating a biomarker response profile based on the biomarker data.

14. Method according to claim 13, comprising the step of determining an anhedonia measure based on the biomarker response profile.

15. A computer program product comprising instructions, which, when executed by the system and any of its components according to any of the claims 1 to 12, cause the system and its respective components to perform the steps of the method according to any of the claims 13 or 14, which method steps are performed by the system and/or the respective components according to the method.
